# EUROPEAN PATENT SPECIFICATION

(11) **EP 3 921 336 B1**
(45) Date of publication and mention of the grant of the patent: **01.03.2023**
(21) Application number: 20708837.8
(22) Date of filing: 29.01.2020
(51) Int. Cl.: C07K 14/605, A61K 38/26, A61K 38/28, A61P 3/08

(54) **GLUCAGON ANALOG AGONISTS AND METHODS OF USING THE SAME**
GLUCAGONANALOGAGONISTEN UND VERFAHREN ZU DEREN VERWENDUNG
AGONISTES ANALOGIQUES DU GLUCAGON ET LEURS PROCÉDÉS D'UTILISATION

(30) Priority: 05.02.2019 US 201962801344 P
(43) Date of publication of application: 15.12.2021
(73) Proprietor: ELI LILLY AND COMPANY, Indianapolis, IN 46206-6288 (US)
(72) Inventor: ALSINA-FERNANDEZ, Jorge, Indianapolis, Indiana 46206-6288 (US); COSKUN, Tamer, Indianapolis, IN 46206-6288 (US); GEISER, Andrea Renee, Indianapolis, Indiana 46206-6288 (US)
(74) Representative: Kent, Lindsey Ruth
(86) International application number: PCT/US2020/015539
(87) International publication number: WO 2020/163125

(56) References cited:
- US-A1- 2013 079 278

## Description

This disclosure generally relates to biology and medicine, and more particularly it relates to glucagon analog agonist (GAA) compounds having improved solubility, improved chemical stability, improved physical stability and/or improved preservative compatibility for pump use when compared to native, human glucagon, as well as relates to pharmaceutical compositions including the same and their therapeutic use in treating hypoglycemia.

Over the past several decades, the prevalence of diabetes has continued to rise. The current standard of care for diabetes includes diet and exercise, as well as treatment with oral medications and injectable glucose-lowering drugs.

Glucagon is a 29-amino acid peptide hormone (SEQ ID NO: 1) secreted by α-cells of the islet of Langerhans in the pancreas and is involved in glucose homeostasis. Under normal physiological conditions, glucagon increases when blood glucose falls, which causes glycogen in the liver to be broken down into glucose for release into the bloodstream. In an individual having diabetes, hypoglycemia can occur as a side effect of diabetes treatment. Moreover, the physiological glucagon response to hypoglycemia in such individuals may be impaired, making it harder for glucose levels to return to the normal range. If left untreated, severe or acute hypoglycemia can cause serious issues such as seizures, unconsciousness, brain damage or even death.

Glucagon is an established therapy for treating acute hypoglycemia. In fact, emergency glucagon administration can restore normal glucose levels within minutes of its administration. Glucagon prepared for administration, however, has several disadvantages. For example, in aqueous buffers at or near physiological pH, glucagon has poor solubility. Likewise, and when formulated at low or high pH, glucagon also demonstrates poor chemical stability and poor physical stability such as gelation and soluble aggregate formation. To minimize these disadvantages, current commercial glucagon-based therapies are provided as a lyophilized powder with instructions to reconstitute at the time of administration. In an emergency situation, reconstituting a lyophilized powder is burdensome and inconvenient. Thus, it is desirable to provide a compound for therapeutic use that maintains the biological performance of glucagon under physiological conditions yet exhibits sufficient aqueous solubility, chemical stability and physical stability under non-physiological conditions.

Glucagon analogs are known that have amino acid substitutions to improve solubility and stability in acidic and physiological pH buffers. *See, e.g.,* Intl. Patent Application Publication Nos. WO 2008/086086, WO 2011/0293586, WO 2015/094875, WO 2015/094876 and WO 2015/094878.

Nevertheless, a need remains for alternative therapeutic agents for treating hypoglycemia, which are capable of providing effective glucose control and which maintain the biological performance of glucagon under physiological conditions while also exhibiting sufficient solubility and chemical and physical stabilities under non-physiological conditions.

To address this need, this disclosure first describes compounds that include an amino acid sequence of:
HSX₁GTFTSDYSKYLD(Aib)RRAQX₂FVK(4-Pal)LLST (Formula I), where X₁ is Q or Dab(Ac) and X₂ is Q or A (SEQ ID NO:2), and where the C-terminal amino acid includes a carboxylic acid at the C-terminus.

In some instances, X₁ is Q and X₂ is Q, such that the compound includes or has an amino acid sequence of:
HSQGTFTSDYSKYLD(Aib)RRAQQFVK(4-Pal)LLST (SEQ ID NO:3), where the C-terminal amino acid includes a carboxylic acid at the C-terminus .

In other instances, X₁ is Q and X₂ is A, such that the compound includes or has an amino acid sequence of:
HSQGTFTSDYSKYLD(Aib)RRAQAFVK(4-Pal)LLST (SEQ ID NO:4), where the C-terminal amino acid includes a carboxylic acid at the C-terminus.

In other instances, X₁ is Dab(Ac) and X₂ is A, such that the compound includes or has an amino acid sequence of:
HS[Dab(Ac)]GTFTSDYSKYLD(Aib)RRAQAFVK(4-Pal)LLST (SEQ ID NO:5), where the C-terminal amino acid includes a carboxylic acid at the C-terminus.

In other instances, X₁ is Dab(Ac) and X₂ is Q, such that the compound includes or has an amino acid sequence of:
HS[Dab(Ac)]GTFTSDYSKYLD(Aib)RRAQQFVK(4-Pal)LLST (SEQ ID NO:6), where the C-terminal amino acid includes a carboxylic acid at the C-terminus.

In yet other instances, the compound is HSQGTFTSDYSKYLD(Aib)RRAQQFVK(4-Pal)LLST (SEQ ID NO:3), HSQGTFTSDYSKYLD(Aib)RRAQAFVK(4-Pal)LLST (SEQ ID NO:4), HS[Dab(Ac)]GTFTSDYSKYLD(Aib)RRAQAFVK(4-Pal)LLST (SEQ ID NO:5), or HS[Dab(Ac)]GTFTSDYSKYLD(Aib)RRAQQFVK(4-Pal)LLST (SEQ ID NO:6), where the C-terminal amino acid includes a carboxylic acid at the C-terminus.

Second, pharmaceutical compositions are described that include at least one of the compounds herein and a pharmaceutically acceptable carrier. In some instances, the pharmaceutically acceptable carrier is a buffer such as, for example, physiological saline, phosphate-buffered saline, citrate-buffered saline or histidine-buffered saline. In certain instances, the buffer is histidine, a histidine buffer or a histidine-buffered saline. In other instances, the pharmaceutical compositions further can include carriers, diluents and/or excipients.

Third, kits are described for administering to an individual at least one compound herein. In some instances, the kits include a syringe and needle for administering the at least one compound. In certain instances, the compound is pre-formulated in an aqueous solution within the syringe. In other instances, the compound is pre-formulated in an aqueous solution in a cartridge for use in a pump setting. In yet other instances, the kits include at least one additional therapeutic agent such as, for example, other antidiabetic agents such as insulin, especially a fast-acting insulin analog, pre-formulated in an aqueous solution in a separate cartridge for use in the pump setting.

Fourth, methods are described for using the compounds herein, especially for using the compounds to treat hypoglycemia. The methods include at least a step of administering to an individual in need thereof an effective amount of at least one compound herein or a pharmaceutically acceptable salt thereof. In some instances, the at least one compound can be administered via any standard route of administration such as, for example, parenterally, intravenously, subcutaneously, intramuscularly or transdermally. In certain instances, the at least one compound is administered subcutaneously (SQ) or intramuscularly (IM).

In particular instances, the at least one compound is SQ administered to the individual as needed *(i.e.,* in response to an acute instance of hypoglycemia). Likewise, and in other instances, the at least one compound can be administered SQ daily (QD), every other day, three times a week, two times a week, one time a week *(i.e.,* weekly; QW), biweekly *(i.e.,* every other week), or monthly. In certain instances, the at least one compound can be administered SQ every other day, SQ three times a week, SQ two times a week, SQ one time a week, SQ every other week, or SQ once a month. In particular instances, the at least one compound is administered QW. Alternatively, and when used in connection with a pump system, the at least one compound can be administered via microdelivery multiple times a day in a chronic setting *(i.e.,* many days in a row). Alternatively still, the at least one compound can be administered in microdoses in a chronic setting using a syringe system *(i.e.,* microinjections in a daily basis).

The methods also can include a step of administering the at least one compound in combination with an effective amount of at least one additional therapeutic agent. In some instances, the at least one additional therapeutic agent can be administered simultaneously, separately or sequentially with the at least one compound.

In some instances, the at least one additional therapeutic agent can be administered with a frequency same as the at least one compound *(i.e.,* daily, every other day, twice a week, weekly or monthly). In other instances, the at least on additional therapeutic agent is administered with a frequency distinct from the at least one compound. The at least one additional therapeutic agent can be administered via any standard route of administration such as, for example, parenterally, intravenously, subcutaneously, intramuscularly or transdermally. In some instances, the route of administration for the at least one additional therapeutic agent can be the same as the at least one compound or can be distinct from the at least one compound.

In some instances, the individual is a person with diabetes (PwD), especially type 1 diabetes mellitus. In other instances, the individual is a person experiencing an acute instance of hypoglycemia *(i.e.,* emergency administration) who may or may not have diabetes.

The methods also may include steps such as measuring or obtaining blood glucose and comparing such obtained values to one or more baseline values or previously obtained values to assess the effectiveness of treatment/therapy.

The methods also may be combined with diet and exercise and/or may be combined with additional therapeutic agents other than those discussed above.

Furthermore, methods are provided for using the compounds herein in a pump system, such as an insulin pump or a bi-hormonal *(e.g.,* insulin-glucagon) pump system.

In view of the above, several uses are provided that include at least one of the compounds herein. For example, the compounds herein can be provided for use in therapy, especially for treating hypoglycemia, which optionally can be provided with at least one additional therapeutic agent for separate, sequential or simultaneous combination with the at least one compound. Likewise, the compounds herein can be provided for use in manufacturing a medicament for treating hypoglycemia, where the medicament optionally may further include at least one additional therapeutic agent.

An advantage of the compounds herein is that they not only maintain wild-type glucagon activity but also exhibit increased aqueous solubility, increased chemical stability, increased physical stability, or reduced fibrillation when compared to native, human glucagon in aqueous solution. Moreover, the compounds herein exhibit similar activity as native, human glucagon *(e.g.,* potency, time of action and selectivity at the glucagon receptor when compared to human glucagon). Furthermore, the compounds herein demonstrate up to about 10x more selectivity than recombinant, human glucagon at the glucagon-like peptide-1 (GLP-1) receptor and no activity at the glucose-dependent insulinotropic polypeptide (GIP) receptor and the glucagon-like peptide-2 (GLP-2) receptor, as well as demonstrate enhanced solubility at a pH in a range of about 5-7. In this manner, the compounds herein are suitable for treating hypoglycemia, including instances of acute hypoglycemia *(i.e.,* emergency administration). The improved properties of the compounds herein also allow for a preparation of glucagon in aqueous solutions for pump administration. Likewise, the compounds can be administered in combination with a fast-acting insulin analog in a dual-chamber pump to provide closed-loop glycemic control. Moreover, the compounds herein have association state and hydrophilicity profiles very similar to native, human glucagon in addition to very similar pharmacokinetic profiles upon subcutaneous administration.

### Definitions and Abbreviations

Reference to an element by the indefinite article "a" or "an" does not exclude the possibility that more than one element is present, unless the context clearly requires that there be one and only one element. The indefinite article "a" or "an" thus usually means "at least one."

As used herein, "about" means within a statistically meaningful range of a value or values such as, for example, a stated concentration, length, molecular weight, pH, sequence identity, time frame, temperature or volume. Such a value or range can be within an order of magnitude typically within 20%, more typically within 10%, and even more typically within 5% of a given value or range. The allowable variation encompassed by "about" will depend upon the particular system under study, and can be readily appreciated by one of skill in the art. "About" therefore is used to indicate that a value includes an inherent variation of error for the device, variation of the method/protocol/technique being employed to determine a value, or even variation that exists among the study individuals.

As used herein, "activity," "activate," "activating" and the like means a capacity of the compounds herein to bind to and induce a response at the receptor, as measured using assays known in the art, such as the *in vitro* assays described below.

As used herein, "amino acid" means a molecule that, from a chemical standpoint, is characterized by the presence of one or more amine groups and one or more carboxylic acid groups, and may contain other functional groups. As is known in the art, there is a set of twenty amino acids which are designated as standard amino acids, and that are used as building blocks for most of the peptides/proteins produced by any living being. The amino acid sequences of in this disclosure contain the standard single letter or three letter codes for the twenty naturally occurring amino acids.

As used herein, "analog" means a compound, such as a synthetic peptide, that activates a target receptor and elicits at least one *in vivo* or *in vitro* effect elicited by a native agonist for that receptor.

As used herein, "effective amount" means an amount, concentration or dose of one or more compounds herein, or a pharmaceutically acceptable salt thereof which, upon single or multiple dose administration to an individual in need thereof, provides a desired effect in such an individual under diagnosis or treatment *(i.e.,* may produce a clinically measurable difference in a condition of the individual such as, for example, an increase in blood glucose, and/or a reduction in weight or body fat). An effective amount can be readily determined by one of skill in the art by using known techniques and by observing results obtained under analogous circumstances. In determining the effective amount for an individual, a number of factors are considered, including, but not limited to, the species of mammal, its size, age and general health, the specific disease or disorder involved, the degree of or involvement or the severity of the disease or disorder, the response of the individual, the particular compound administered, the mode of administration, the bioavailability characteristics of the preparation administered, the dose regimen selected, the use of concomitant medication, and other relevant circumstances.

As used herein, "fibrillation" means gelation and soluble aggregate formation observed when glucagon is formulated at a low or high pH.

As used herein, "half-maximal effective concentration" or "EC₅₀" means a concentration of compound that results in 50% activation/stimulation of an assay endpoint, such as a dose-response curve *(e.g.,* cAMP).

As used herein, "in combination with" means administering at least one of the compounds herein either simultaneously, sequentially or in a single combined formulation with one or more additional therapeutic agents.

As used herein, "glucagon analog agonist" or "GAA" means a compound having structural similarities with, but multiple differences from, glucagon, especially native, human glucagon (SEQ ID NO: 1) or recombinant, human glucagon. The compounds herein include amino acid sequences resulting in the compounds having affinity for and activity at the glucagon receptor.

As used herein, "hypoglycemia" means a blood glucose below about 72 mg/dL (about 4 mmol/L).

As used herein, "individual in need thereof' means a mammal, such as a human, with a condition, disease, disorder or symptom requiring treatment or therapy, including for example, those listed herein.

As used herein, "non-standard amino acid" means an amino acid that may occur naturally in cells but does not participate in peptide synthesis. Non-standard amino acids can be constituents of a peptide and often times are generated by modification of standard amino acids in the peptide *(i.e.,* via post-translational modification). Non-standard amino acids can include D-amino acids, which have an opposite absolute chirality of the standard amino acids above. Herein, "Aib" is alpha amino isobutyric acid, "Dab(Ac)" is 2,4-diaminobutryic acid (Dab) with an acetyl group (Ac) forming an amide bond with the amino group at position 4, and "4-Pal" is 3-(4-pyridyl)-L-alanine/4-pyridyl-L-alanine.

As used herein, "pharmaceutically acceptable buffer" means any of the standard pharmaceutical buffers known to one of skill in the art.

As used herein, "treating" or "to treat" means attenuating, restraining, reversing, slowing or stopping progression or severity of an existing condition, disease, disorder or symptom.

Certain abbreviations are defined as follows: "ACN" refers to acetonitrile; "ACR" refers to urine albumin/urine creatinine ratio; "amu" refers to atomic mass unit; "cAMP" refers to cyclic adenosine monophosphate; "CRC" refers to complete response curve; "DIC" refers to diisopropylcarbodiimide; "DMF" refers to dimethylformamide; "DMSO" refers to dimethyl sulfoxide; "EDTA" refers to ethylenediaminetetraacetic acid; "EIA/RIA" refers to enzyme immunoassay/radioimmunoassay; "Fmoc" refers to fluorenylmethoxycarbonyl; "hr" refers to hour(s); "HEPES" refers to 4-(2-hydroxyethyl)-1-piperazineethanesulfonic acid; "HOBt" refers to hydroxybenzotriazole; "HTRF" refers to homogenous time-resolved fluorescent; "IV" refers to intravenous; "kDa" refers to kilodaltons; "LC-MS" refers to liquid chromatography-mass spectrometry; "min" refers to minute(s); "MS" refers to mass spectrometry; "OtBu" refers to O-tert-butyl; "Pbf" refers to NG-2,2,4,6,7-pentamethyldihydrobenzofuran-5-sulfonyl; "RP-HPLC" refers to reversed-phase high performance liquid chromatography; "SQ" refers to subcutaneous; "SEM" refers to standard error of the mean; "tBoc" refers to tert-butoxycarbonyl; "TFA" refers to trifluoroacetic acid; "Trt" refers to trityl; and "WGA" refers to wheat germ agglutinin.

### Glucagon Analog Agonists

The compounds herein have structural differences from native, human glucagon (SEQ ID NO: 1). For example, the compounds herein include modifications at one or more of positions 3, 16, 21, 24, 25, 27 and 28 with respect to the numbering of native, human glucagon (SEQ ID NO:1). Exemplary amino acid sequences of the compounds herein include (specific changes relative to corresponding residue of native, human glucagon (SEQ ID NO:1) are in bold):
HSX₁GTFTSDYSKYLD**(Aib)**RRAQ**X₂**FVK**(4-Pal)**L**LS**T (SEQ ID NO:2);
HSQGTFTSDYSKYLD(Aib)RRAQQFVK**(4-Pal)LLST** (SEQ ID NO:3);
HSQGTFTSDYSKYLD**(Aib)**RRAQAFV**K(4-Pal)LLST** (SEQ ID NO:4);
**HS[Dab(Ac)]GTFTSDYSKYLD(Aib)RRAQAFVK(4-Pal)LLST** (SEQ ID NO:5); and
**HS[Dab(Ac)]GTFTSDYSKYLD(Aib)RRAQQFVK(4-Pal)LLST** (SEQ ID NO:6), where the C-terminal amino acid of each may include a carboxylic acid at the C-terminus.

Moreover, and to improve *in vivo* compatibility and effectiveness, the compounds herein may be reacted with any of a number of inorganic and organic acids/bases to form pharmaceutically acceptable acid/base addition salts. Pharmaceutically acceptable salts and common techniques for preparing them are well known in the art (*see, e.g.,* Stahl et al., "Handbook of Pharmaceutical Salts: Properties, Selection and Use," 2nd Revised Edition (Wiley-VCH, 2011)). Pharmaceutically acceptable salts for use herein include sodium, trifluoroacetate, hydrochloride and acetate salts.

Half-life of the compounds herein may be measured using techniques known in the art including, for example, those described in the Examples below. Likewise, affinity of the compounds herein for the glucagon receptor may be measured using techniques known in the art for measuring receptor binding levels including, for example, those described in the Examples below, and is commonly expressed as an inhibitory constant (Kᵢ) value. Moreover, activity of the compounds herein at the glucagon receptor may be measured using techniques known in the art, including, for example, those described in the Examples below, and is commonly expressed as an EC₅₀ value.

The compounds herein can be formulated as pharmaceutical compositions, which can be administered by parenteral routes (*e.g.,* intravenous, intraperitoneal, intramuscular, subcutaneous or transdermal). Such pharmaceutical compositions and techniques for preparing the same are well known in the art. *See, e.g.,* Remington, "The Science and Practice of Pharmacy" (D.B. Troy ed., 21st Edition, Lippincott, Williams & Wilkins, 2006). In particular instances, the compounds herein are administered SQ or IM. Alternatively, however, the compounds may be formulated in forms for other pharmaceutically acceptable routes such as, for example, tablets or other solids for oral administration; time release capsules; and any other form currently used, including creams, lotions, inhalants and the like.

The compounds herein may be administered by a physician or self-administered using an injection. It is understood that one of skill in the art can readily determine the gauge size and amount of injection volume. However, the amount of injection volume can be ≤ about 2 mL or even ≤ about 1 mL, and the needle gauge can be ≥ about 27 G or even ≥ about 29 G.

The disclosure also provides and therefore encompasses novel intermediates and methods useful for synthesizing the compounds herein, or a pharmaceutically acceptable salt thereof. The intermediates and compounds herein can be prepared by a variety of techniques that are well known in the art. For example, a method using chemical synthesis is illustrated in the Examples below. The specific synthetic steps for each of the routes described may be combined in different ways to prepare the compounds herein. The reagents and starting materials are readily available to one of skill in the art.

In this manner, the pharmaceutical composition can include an effective amount of a compound having SEQ ID NO:2 and a pharmaceutically acceptable carrier, an effective amount of a compound having SEQ ID NO:3 and a pharmaceutically acceptable carrier, an effective amount of a compound having SEQ ID NO:4 and a pharmaceutically acceptable carrier, an effective amount of a compound having SEQ ID NO:5 and a pharmaceutically acceptable carrier, or an effective amount of a compound having SEQ ID NO:6 and a pharmaceutically acceptable carrier, or even combinations thereof and a pharmaceutically acceptable carrier.

The compounds herein are generally effective over a wide dosage range. Exemplary doses of the compounds herein or of pharmaceutical compositions including the same can be milligram (mg) or microgram (µg) or picogram (pg) amounts per kilogram (kg) of an individual. In this manner, a daily dose can be from about 1 µg to about 100 mg.

Here, the effective amount of the compounds herein in a pharmaceutical composition can be a dose of about 0.01 mg to about 10 mg, of about 0.1 mg to about 3 mg, or of about 0.01 mg to about 0.03 mg. One of skill in the art, however, understands that in some instances the effective amount *(i.e.,* dose/dosage) may be below the lower limit of the previously mentioned range and be more than adequate, while in other cases the effective amount may be a larger doses and may be employed with acceptable side effects.

Moreover, the pharmaceutical composition can have a pH that is physiologically acceptable. In some instances, the pH can range from about 4 to about 8, or from about 5 to about 6.

In addition to at least one of the compounds herein, the pharmaceutical composition can include one or more additional therapeutic agents, especially other antidiabetic or weight loss agents. In some instances, the additional therapeutic agent can be an insulin, especially a fast-acting insulin such as Humalog^{®} (Eli Lilly and Company; Indianapolis, IN).

The compounds herein can be provided as part of a kit. In some instances, the kit includes a device for administering at least one compound herein (and optionally at least one additional therapeutic agent) to an individual. In certain instances, the kit includes a syringe and needle for administering the at least one compound herein (and optionally at least one additional therapeutic agent). In particular instances, the at least one compound herein (and optionally at least one additional therapeutic agent) is pre-formulated in aqueous solution within the syringe.

Alternatively, the compounds herein can be used in a pump system, such as an insulin pump or a bi-hormonal (*e.g.,* insulin-glucagon) pump system.

The compounds herein can be synthesized via any number of peptide synthesis techniques known in the art using standard manual or automated solid-phase synthesis procedures. Automated peptide synthesizers are commercially available from, for example, Applied Biosystems (Foster City, CA) and Protein Technologies Inc. (Tucson, AZ). Reagents for solid-phase synthesis are readily available from commercial sources. Solid-phase synthesizers can be used according to the manufacturer's instructions for blocking interfering groups, protecting amino acids during reaction, coupling, deprotecting and capping of unreacted amino acids.

Typically, an N-α-carbamoyl-protected amino acid and the N-terminal amino acid on the growing peptide chain attached to a resin are coupled at room temperature in an inert solvent such as DMF, N-methylpyrrolidone or methylene chloride in the presence of coupling agents such as diisoprop1-carbodiimide and 1-hydroxybenzotriazole. The Nα-carbamoyl protecting group is removed from the resulting peptide resin using a reagent such as TFA or piperidine, and the coupling reaction is repeated with the next desired Nα-protected amino acid to be added to the peptide chain. Suitable amine protecting groups are well known in the art and are described, for example, in Green & Wuts, "Protecting Groups in Organic Synthesis," (John Wiley and Sons, 1991). The most commonly used examples include tBoc and Fmoc. After completion of synthesis, peptides are cleaved from the solid-phase support with simultaneous side chain deprotection using standard treatment methods under acidic conditions.

One of skill in the art will appreciate that the peptide chains described herein can be synthesized with a C-terminal carboxylic acid. For the synthesis of C-terminal carboxylic acid peptides, Wang resins and chloro-trityl resins can be used with Fmoc synthesis.

Crude peptides typically are purified using RP-HPLC on C8 or C18 columns using water-ACN gradients in 0.05 % to 0.1 % TFA. Purity can be verified by analytical RP-HPLC. Identity of peptides can be verified by MS. Peptides can be solubilized in aqueous buffers over a wide pH range.

### Methods of Using the Glucagon Analog Agonists

One use of the compounds herein is as an adjunct to insulin to improve glycemic control in individuals, especially in individuals who have diabetes. In this regard, administering a compound herein can result in glycemic control by rapidly attenuating an acute instance of hypoglycemia that may have been brought on by an administration or release of too much insulin. Alternatively, and in individuals not having diabetes, hypoglycemia can be brought about by certain medications *(e.g.,* quinine), excessive alcohol consumption, pregnancy, a disorder affecting the liver *(e.g.,* hepatitis), heart or kidneys, an eating disorder such as anorexia, a tumor of the pancreas that causes too much insulin *(e.g.,* insulinoma or nesidioblastosis), and even certain hormone deficiencies resulting from disorders of the adrenal glands or pituitary gland. It therefore is contemplated that the compounds herein can be used to treat hypoglycemia in these instances as well.

The methods can include steps as described herein the may be, but not necessarily, carried out in the sequence as described. Other sequences, however, also are conceivable. Moreover, individual or multiple steps may be carried out either in parallel and/or overlapping in time and/or individually or in multiply repeated steps. Furthermore, the methods may include additional, unspecified steps.

Such methods therefore can include selecting an individual who has diabetes or is predisposed to the same. Alternatively, the methods can include selecting an individual who has diabetes or is predisposed to the same and who is experiencing an acute instance of hypoglycemia. Alternatively still, the methods can include selecting an individual who does not have diabetes but who is experiencing an acute instance of hypoglycemia.

The methods also can include administering to the individual an effective amount of at least one compound herein, which may be in the form of a pharmaceutical composition as described herein. In some instances, the at least one compound/pharmaceutical composition can include one or more additional therapeutic agents such as an insulin, especially a fast-acting insulin analog.

The concentration/dose/dosage of the at least one compound and optional additional therapeutic agent are discussed elsewhere herein.

With regard to a route of administration, the at least one compound or pharmaceutical composition including the same can be administered in accord with known methods such as, for example, orally, by injection *(i.e.,* intra-arterially, intravenously, intraperitoneally, intracerebrally, intracerebroventricularly, intramuscularly, intraocularly, intraportally or intralesionally), by sustained release systems, or by implantation devices. In certain instances, the at least one compound or pharmaceutical composition including the same can be administered SQ by bolus injection *(e.g.,* QD or QW) or continuously (including by microdosing or microinfusion). In other instances, the at least one compound or pharmaceutical composition including the same can be administered IM by bolus injection.

With regard to a dosing frequency, the at least one compound or pharmaceutical composition including the same can be administered as needed for an acute instance of hypoglycemia. Alternatively, the at least one compound or pharmaceutical composition including the same can be administered daily, every other day, three times a week, two times a week, one time a week *(i.e.,* weekly), biweekly *(i.e.,* every other week), or monthly. In certain instances, the at least one compound or pharmaceutical composition including the same is administered SQ every day (QD), SQ every other day, SQ three times a week, SQ two times a week, SQ one time a week (QW), SQ every other week or SQ monthly (QM). In particular instances, the at least one compound or pharmaceutical composition including the same is administered QD or QW. In other instances, the at least one compound or pharmaceutical composition including the same is administered IM every day, IM every other day, IM three times a week, IM two times a week, IM one time a week, IM every other week or IM monthly.

With regard to those instances in which the at least one compound or pharmaceutical composition including the same is used in combination with an effective amount of an insulin, the insulin can be a fast-acting insulin, which can be administered simultaneously, separately or sequentially with the at least one compound or pharmaceutical composition including the same. In this instance, the at least one compound and the fast-acting insulin are pre-formulated in an aqueous solution in separate cartridges for use in a pump setting.

In this manner, the insulin can be administered with a frequency same as the at least one compound or pharmaceutical composition including the same *(i.e.,* every other day, twice a week, or even weekly). Alternatively, insulin can be administered with a frequency distinct from the at least one compound or pharmaceutical composition including the same. Furthermore, the insulin can be administered via the same or a distinct route as the at least one compound *(e.g.,* both SQ; one SQ, the other orally; one SQ, the other IM, *etc.).*

It is further contemplated that the methods may be combined with diet and exercise and/or may be combined with additional therapeutic agents other than those discussed above.

### EXAMPLES

The following non-limiting examples are offered for purposes of illustration, not limitation.

### PEPTIDE SYNTHESIS

### Example 1:

Example 1 is a compound having an amino acid sequence of:
HSQGTFTSDYSKYLD(Aib)RRAQQFVK(4-Pal)LLST-OH (SEQ ID NO:3).

Structurally, the compound of SEQ ID NO:3 is as follows: 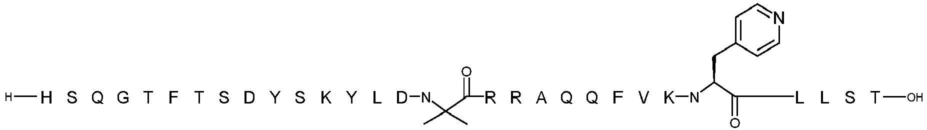 , where the above structure recites the standard single letter amino acid code with the exception of Aib at position 16 and 4-Pal at position 25, which are expanded.

Here, the compound of SEQ ID NO:3 is generated by solid-phase peptide synthesis on a Protein Technologies Inc. Symphony Instrument. Synthesis (0.125 mmols scale) starts from Fmoc-Thr-(tBu)-Wang resin (Advanced Chem Tech) with substitution of about 0.32 mmol/g. The synthesis uses a Fmoc/tBu protecting group strategy. Amino acid side-chain derivatives are: Arg (Pbf), Asp(OtBu), Gln(Trt), Glu(OtBu), His(Trt), Lys(Boc), Ser(OtBu), Thr(OtBu), Trp(Boc) and Tyr(OtBu). Coupling is carried out with about 10 equivalents of amino acid activated with DIC and ethyl cyanohydroxyiminoacetate (Oxyma) (1:1:1 molar ratio) in DMF. Coupling is carried out for 3 hr at room temperature.

Concomitant cleavage from the resin and side chain protecting group removal is carried out in a solution containing TFA: triisopropylsilane : 1,2-ethanedithiol : water : thioanisole 90:4:2:2:2 (v/v) for 2 hr at room temperature. The solution is filtered, and peptide is precipitated with cold diethyl ether and centrifuged at 4000 rpm for 2 min (cold ether washing repeated for three times).

Crude peptide is dissolved in 40 mL of water containing 10% acetic acid and purified on a C18 RP-HPLC column (Waters SymmetryPrep 7 µm, 19 × 300 mm) at a flow rate of 18 mL/min. Sample is eluted with a linear AB gradient of 16%-36% B over 90 min, where A = 0.1% TFA in water and B = ACN. Product elutes at about 25% B. The TFA salt is converted to the acetate salt by diluting combined TFA fractions to 2x the volume in 0.1% TFA in water. Material is loaded onto the column followed by 50 mL of water loaded onto column, and then 250 mL of 0.1 M ammonium acetate solution and an additional 50 mL of water. Sample is eluted with a linear AB gradient of 5%-20% B over 75 min, where A = 2 % acetic acid in water and B = ACN. Product elutes at about 18% B. The pure fractions are combined and lyophilized.

Peptide purity and molecular weight (MW) is confirmed on an Agilent 1240 Infinity II LC-MS System with a single quadrupole MS detector. Analytical HPLC separation is done on an Acquity UPLC RP18, 1.7 µm, 2.1 mm × 100 mm column with a linear AB gradient of 10%-40% B over 20 min in which A = 0.05% TFA in water and B = 0.05% TFA in ACN and the flow rate is 0.5 mL/min (wavelength of 220 nm). The compound is purified to > 98 % purity and is confirmed to have MW corresponding to the calculated value within 1 atomic mass unit (amu). MW=3410.8; Calculated: M+2H⁺/2=1706.3, M+3H⁺/3=1137.9, M+4H⁺/4=853.7; Found: M+2H⁺/2=1706.4, M+3H⁺/3=1137.8, M+4H⁺/4=853.7.

### Example 2:

Example 2 is a compound having a structure of:
HSQGTFTSDYSKYLD(Aib)RRAQAFVK(4-Pal)LLST-OH (SEQ ID NO:4).

Structurally, the compound of SEQ ID NO:4 is as follows: 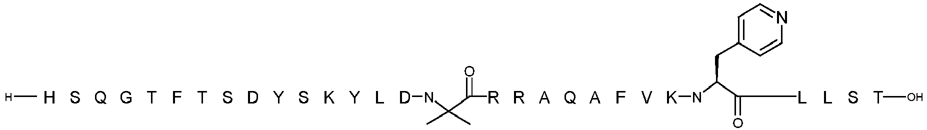 , where the above structure recites the standard single letter amino acid code with the exception of Aib at position 16 and 4-Pal at position 25, which are expanded.

Here, the compound of SEQ ID NO:4 is generated essentially as described for Example 1. After cleavage from the resin and concomitant removal of the side-chain protecting groups, crude peptide is purified as described in Example 1 using a linear AB gradient of 16%-36% B over 90 min, where A = 0.1% TFA in water and B = ACN. Product elutes at about 24% B. TFA salt is converted to acetate salt as described in Example 1, using a linear AB gradient of 5%-20% B over 75 min, where A = 2% acetic acid in water and B = ACN. Product elutes at about 17% B. Pure fractions are combined and lyophilized.

Characterization of the purified peptide is carried out as described in Example 1 and confirmed to have MW corresponding to the calculated value within 1 atomic mass unit (amu). MW=3353.8; Calculated: M+2H⁺/2=1677.8, M+3H⁺/3=1118.9, M+4H⁺/4=839.4; Found: M+2H⁺/2=1677.8, M+3H⁺/3=1118.8, M+4H⁺/4=839.3.

### Example 3:

Example 3 is a compound having a structure of:
HS[Dab(Ac)]GTFTSDYSKYLD(Aib)RRAQAFVK(4-Pal)LLST-OH (SEQ ID NO:5).

Structurally, the compound of SEQ ID NO:5 is as follows: 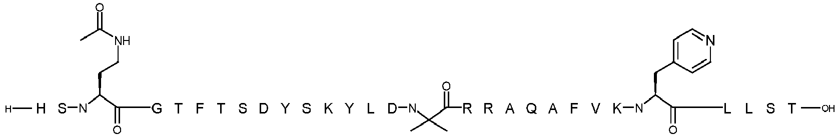 , where the structure recites the standard single letter amino acid code with exception the of Dab(Ac) at position 3, Aib at position 16, and 4-Pal at position 25, which are expanded.

Here, the compound of SEQ ID NO:5 is generated essentially as described in Example 1. Fmoc-Dab(Alloc)-OH building block is used for Dab3 coupling (orthogonal protecting group) to allow for site specific acetylation later on in the synthetic process. The N-terminal residue is incorporated as Boc-His(Trt)-OH using same protocols as described in Example 1. After finishing the elongation of the peptide-resin, the Alloc protecting group present in Dab3 is removed using catalytic amounts of Pd(PPh₃)₄ in the presence of PhSiH₃ as a scavenger. Acetylation at Dab3 position is achieved using acetic acid activated with DIC and Oxyma as described in Example 1. After cleavage from the resin and concomitant removal of the side-chain protecting groups, crude peptide is purified as described in Example 1 using a linear AB gradient of 16%-36% B over 90 min, where A = 0.1% TFA in water and B = ACN. Product elutes at about 25% B. TFA salt is converted to acetate salt as described in Example 1, using a linear AB gradient of 5%-20%B over 75 min, where A = 2% acetic acid in water and B = ACN. Product elutes at about 20% B. Pure fractions are combined and lyophilized.

Characterization of the purified peptide is carried out as described in Example 1 and confirmed to have MW corresponding to the calculated value within 1 atomic mass unit (amu). MW=3367.8; Calculated: M+2H⁺/2=1684.8, M+3H⁺/3=1123.5, M+4H⁺/4=842.9; Found: M+2H⁺/2=1684.8, M+3H⁺/3=1123.4, M+4H⁺/4=842.9.

### Example 4:

Example 4 is a compound having a structure of:
HS[Dab(Ac)]GTFTSDYSKYLD(Aib)RRAQQFVK(4-Pal)LLST-OH (SEQ ID NO:6).

Structurally, the compound of SEQ ID NO:6 is as follows: 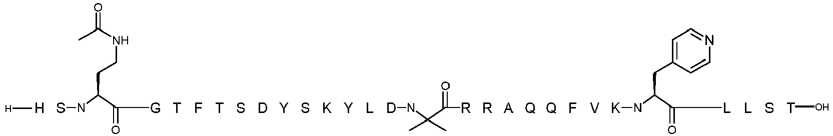 , where the structure recites the standard single letter amino acid code with the exception of Dab(Ac) at position 3, Aib at position 16, and 4-Pal at position 25, which are expanded.

Here, the compound of SEQ ID NO:6 is generated essentially as described in Example 3. After cleavage from the resin and concomitant removal of the side-chain protecting groups, crude peptide is purified as described in Example 1 using a linear AB gradient of 16%-36% B over 90 min, where A = 0.1% TFA in water and B = ACN. Product elutes at about 26% B. TFA salt is converted to acetate salt as described in Example 1, using a linear AB gradient of 5%-20%B over 75 min, where A = 2% acetic acid in water and B = ACN. Product elutes at about 19% B. Pure fractions are combined and lyophilized.

Characterization of the purified peptide is carried out as described in Example 1 and confirmed to have MW corresponding to the calculated value within 1 atomic mass unit (amu). MW=3424.8; Calculated: M+2H⁺/2=1713.4; M+3H⁺/3=1142.6, M+4H⁺/4=857.2; Found: M+2H⁺/2=1713.4; M+3H⁺/3=1142.4, M+4H⁺/4=857.2.

### IN VITRO FUNCTION

### Example 5: Solubility and Chemical Stability of the Compounds of Examples 1-4

### Methods

The compounds of Examples 1-4 are prepared in H5.5PG/mCresol buffer (10 mM Histidine buffer pH5.5, 19 mg/mL propylene glycol, 3.15 mg/mL mCresol), H6PG/mCresol buffer (10 mM Histidine buffer pH6, 19 mg/mL propylene glycol, 3.15 mg/mL mCresol) and H6.5PG/mCresol buffer (10 mM Histidine buffer pH6.5, 19 mg/mL propylene glycol, 3.15 mg/mL mCresol). The final concentration for the compound of Example 2 is 5 mg/mL and the compounds of Examples 1, 3 and 4 is 2 mg/mL. All solutions are filtered through a 0.22 µm filter (Millex, SLGV004SL), and transferred to several HPLC vials (DWK LIFE SCIENCES INC, Wheaton 0.3 mL vial, catalog number 225326, cap catalog number W22533001). Samples are then maintained at 4°C and 37°C. Samples are visually assessed at different time points for turbidity and phase separation.

Stability of the compounds are assessed by RP-HPLC on a Phenomenex Aeris Widepore, 3.6 µm, XB-C18 4.6 × 100 mm column (P/NO 00D-4482-E0) heated at 60°C with a AB (A = 0.05 % TFA/H₂O; B = 0.04 % TFA/ACN) gradient of 5 % B isocratic over 5 min, 5%-25 % B over 20 min, 25%-30 % B over 30 min, and 30%-45 % B over 10 min with a flow rate of 1.2 mL/min (wavelength of 220 nm).

### Results

The compounds maintain solubility at 4°C and 37°C at pH 5.5 (Buffer H5.5PG/mCresol), pH 6 (Buffer H6PG/mCresol) and pH6.5 (Buffer H6.5PG/mCresol) over 4 weeks both by visual assessment and by RP-HPLC. Physical appearance is clear to colorless, with no opalescences and no particles. Recovery by RP-HPLC is shown below in Table 1.

**Table 1:**

| Compound | Buffer | Total Peak Area day 0 | 4°C Total Peak Area 4wk | 37°C Total Peak Area 4wk | 37°C vs 4°C Recovery 4wk |
|---|---|---|---|---|---|
| Example 1 (2 mg/mL) | H5.5PG/ mCresol | 7584621 | 7578303 | 7540348 | 99% |
| | H6.0PG/ mCresol | 7606571 | 7610787 | 7557864 | 99% |
| | H6.5PG/ mCresol | 7593441 | 7562672 | 7513681 | 99% |
| Example 2 (5 mg/mL) | H5. 5PG/ mCresol | 7121811 | 7320325 | 7178483 | 98% |
| | H6.0PG/ mCresol | 6968526 | 7109756 | 7250974 | 102 % |
| | H6.5PG/ mCresol | 6935826 | 7252320 | 7268393 | 100% |
| Example 3 (2 mg/mL) | H5.5PG/ mCresol | 7312638 | 7506703 | 7426077 | 99% |
| | H6.0PG/ mCresol | 7267109 | 7446442 | 7482277 | 100% |
| | H6.5PG/ mCresol | 7214203 | 7398069 | 7366577 | 100% |
| Example 4 (2 mg/mL) | H5.5PG/ mCresol | 7512111 | 7503580 | 7382346 | 98 % |
| | H6.0PG/ mCresol | 7525646 | 7489118 | 7430403 | 99% |
| | H6.5PG/ mCresol | 7421433 | 7362230 | 7325053 | 99% |

As shown below in Table 2, assessment of the compounds by RP-HPLC indicates main peak changes of less than 4.5 % in Buffer H5.5PG/mCresol (pH 5.5 at 4-wk 37°C vs T0 and in Buffer H6PG/mCresol (pH 6 at 4-wk 37°C vs T0; ≤5.32% in Buffer H6.5PG/mCresol (pH 6.5 at 4-wk 37°C vs T0).

**Table 2:**

| Compound | Buffer | % Main Peak Day 0 | 4°C % Main Peak | 37°C % Main Peak |
|---|---|---|---|---|
| Example 1 (2 mg/mL) | H5.5PG/ mCresol | 99.83 | 99.69 | 95.43 |
| | H6.0PG/ mCresol | 99.78 | 99.49 | 95.48 |
| | H6.5PG/ mCresol | 99.52 | 99.58 | 95.06 |
| Example (5 mg/mL) | H5. 5PG/ mCresol | 99.78 | 99.69 | 95.21 |
| | H6.0PG/ mCresol | 99.93 | 99.85 | 95.75 |
| | H6.5PG/ mCresol | 99.80 | 99. .68 | 94.48 |
| Example 3 (2 mg/mL) | H5.5PG/ mCresol | 99.22 | 99.06 | 95.26 |
| | H6.0PG/ mCresol | 99.23 | 99.26 | 94.94 |
| | H6.5PG/ mCresol | 99.11 | 99.25 | 94.72 |
| Example 4 (2 mg/mL) | H5.5PG/ mCresol | 98.84 | 98.49 | 94.48 |
| | H6.0PG/ mCresol | 98.71 | 98.71 | 94.76 |
| | H6.5PG/ mCresol | 98.76 | 98.86 | 94.51 |

### Example 6: Physical Stability of the Compounds of Examples 1-4

### Methods

A Thioflavin T binding assay is performed to assess the level of fibrillation of the compounds. The compounds of Examples 1-4 are dissolved in H6PG/mCresol buffer (Example 2 at 5 mg/mL, and Examples 1, 3 and 4 at 2 mg/mL) and filled in a pump bag made of COP-Coex (Zacros Q4 2017) material to prevent evaporation. The samples in bag are stored at 4°C and 37°C with and without agitation (150 rpm, linear direction) for 2 weeks.

Aliquots of the different samples (100 µL each aliquot and done in triplicates) are taken at time points 0 and 2 weeks, which then are added to a plate followed by 10 µL of a 1 mM Thioflavin T (stock solution in H₂O, pH 2.8) (T35516-25G, Sigma Aldrich). Samples are incubated for 30 min. Fluorescence is measured using a Spectramax M5 (Moleculer Devices) using 440 ηm as the excitation wavelength, and the emission wavelength is set at 480 ηm with a 475 ηm cut off and automatic sensitivity adjustment. Raw data is collected with Softmax Pro 5.4.1 (Molecular Devices) and imported to Excel^{®}. The average of the 3 wells per each time point becomes the reported fluorescence units shown in Table 3 below.

### Results

**Table 3: Physical Stability of the Compounds of Examples 1-4.**

| Sample H6PG/mCresol buffer | | t = 0 hr | t = 2 weeks |
|---|---|---|---|
| Example 1 (2 mg/mL) | 4°C | 11 | 14 |
| | 37°C | | 11 |
| | 37°C/150 rpm | | 14 |
| Example 2 (5 mg/mL) | 4°C | 13 | 15 |
| | 37°C | | 17 |
| | 37°C/150 rpm | | 15 |
| Example 3 (2 mg/mL) | 4°C | 12 | 16 |
| | 37°C | | 15 |
| | 37°C/150 rpm | | 16 |
| Example 4 (2 mg/mL) | 4°C | 12 | 13 |
| | 37°C | | 13 |
| | 37°C/150 rpm | | 14 |
| H6PG/mCresol | | 12 | 10 |

As shown in Table 3, the compounds of Examples 1-4 maintain physical stability at 37°C and pH 6 for two weeks in the presence of agitation stress as assessed by both visual assessment and Thioflavin T binding assay. Moreover, the compounds of Examples 1-4 did not demonstrate fibrillation as measured by the Thioflavin T binding assay.

### Example 7: Association State of the Compounds of Examples 1-4

### Methods

The compounds of Examples 1-4 are prepared in H5.5PG/mCresol buffer (10 mM Histidine buffer pH5.5, 19 mg/mL propylene glycol, 3.15 mg/mL mCresol), H6PG/mCresol buffer (10 mM Histidine buffer pH6, 19 mg/mL propylene glycol, 3.15 mg/mL mCresol) and H6.5PG/mCresol buffer (10 mM Histidine buffer pH6.5, 19 mg/mL propylene glycol, 3.15 mg/mL mCresol). The final concentration for Example 2 is 5 mg/mL, and the final concentration for Examples 1, 3 and 4 is 2 mg/mL. All solutions are filtered through a 0.22 µm filter (Millex, SLGV004SL). Analytical ultracentrifugation (Beckman, model - Xli and model Optima) is used to collect sedimentation velocity data at 60,000 rpm at 20°C for ~ 500 runs (interval = 2 min) by interference detection.

### Results

As shown in Table 4 below, the compounds contain a mixture of monomer, trimer and higher molecular weight species (HMWS).

**Table 4: Association States of the Compounds of Examples 1-4.**

| | Example 1 (2 mg/mL) H5.5PG/mCresol | Example 1 (2 mg/mL) H6PG/mCresol | Example 1 (2 mg/mL) H6.5PG/mCresol |
|---|---|---|---|
| Monomer % | 95.1 | 91.9 | 92.6 |
| Trimer % | 4.2 | 7.0 | 6.6 |
| HMWS % | 0.7 | 1.1 | 0.8 |

| | Example 2 (5 mg/mL) H5.5PG/mCresol | Example 2 (5 mg/mL) H6PG/mCresol | Example 2 (5 mg/mL) H6.5PG/mCresol |
|---|---|---|---|
| Monomer % | 56.0 | 46.2 | 43.9 |
| Trimer % | 44.0 | 53.8 | 56.1 |
| HMWS % | 0 | 0 | 0 |

| | Example 3 (2 mg/mL) H5.5PG/mCresol | Example 3 (2 mg/mL) H6PG/mCresol | Example 3 (2 mg/mL) H6.5PG/mCresol |
|---|---|---|---|
| Monomer % | 89.5 | 84.8 | 84.5 |
| Trimer % | 10.1 | 14.7 | 15.1 |
| HMWS % | 0.4 | 0.6 | 0.4 |

| | Example 4 (2 mg/mL) H5.5PG/mCresol | Example 4 (2 mg/mL) H6PG/mCresol | Example 4 (2 mg/mL) H6.5PG/mCresol |
|---|---|---|---|
| Monomer % | 96.6 | 92.3 | 94.0 |
| Trimer % | 2.9 | 6.6 | 5.4 |
| HMWS % | 0.5 | 1.1 | 0.6 |

The compounds show a slightly higher % monomer at pH 5.5 than at pH 6 and pH 6.5 by analytical centrifugation. At 2 mg/mL, the compounds of Examples 1, 3 and 4 are highly monomeric. % monomer is reduced for the compound of Example 2 because of higher concentration of the test article (5 mg/mL). Overall, the data indicates high monomeric content for the compounds of Examples 1-4, which may result in a faster absorption rate after SQ administration in animal models and humans.

### Example 8: Hydrophilicity Data of the Compounds of Examples 1-4

### Methods

Hydrophilicity of the compounds of Examples 1-4 is assessed by analytical RP-HPLC on a Phenomenex Aeris Widepore, 3.6 µm, XB-C18 4.6 x 100 mm column (P/NO 00D-4482-E0) heated at 60°C with a AB (A = 0.05% TFA/water; B = 0.04% TFA/ACN) gradient of 5% B isocratic over 5 min, 5%-25% B over 20 min, 25%-30% B over 30 min, and 30%-45% B over 10 min with a flow rate of 1.2 mL/min (wavelength of 220 nm).

### Results

As shown in Table 5 below, the compounds have similar or slightly faster retention time by RP-HPLC than human glucagon, which indicates similar or slightly better hydrophilicity profile than human glucagon (i.e., faster retention time indicates higher hydrophilicity).

**Table 5: Retention Time of the Compounds of Examples 1-4 and Native Glucagon.**

| Compound | Retention Time (min) |
|---|---|
| Example 1 | 11.24 |
| Example 2 | 11.50 |
| Example 3 | 11.20 |
| Example 4 | 11.48 |
| Native glucagon | 12.56 |

### Example 9: Binding Affinity of the Compounds of Examples 1-4

### Methods

1. Human Glucagon Receptor Binding Assay: the binding of the compounds of Examples 1-4 is determined by using a 293-HEK cell line overexpressing the human glucagon receptor (hGCGR; *see, e.g.,* Lok et al. (1994) Gene 140:203-209 (1994); *see also,* GenBank Accession No. L20316 for the nucleotide sequence).

Crude plasma membranes are prepared using cells from adherent culture. Frozen cell pellets are lysed on ice in hypotonic buffer containing 50 mM Tris HCl, pH 7.5, and cOmplete^{™} protease inhibitors (Roche Diagnostics GmbH; Mannheim, DE) with EDTA. The cell suspension is homogenized using a glass Potter-Elvehjem homogenizer fitted with a Teflon^{®} pestle for 25 strokes. The homogenate is centrifuged at 4°C at 1100 x g for 10 min. The supernatant is collected and stored on ice, and the pellets are re-suspended in homogenization buffer and re-homogenized. The homogenate is centrifuged at 1100 x g for 10 min. The second supernatant is combined with the first supernatant and centrifuged at 35000 x g for 1 hr at 4°C. The resulting membrane pellet is re-suspended in homogenization buffer containing protease inhibitors at about 1 to 3 mg/mL, quick frozen in liquid nitrogen, and stored as aliquots in a -80°C freezer until use.

Human glucagon (SEQ ID NO:1) is radioiodinated by ¹²⁵I-lactoperoxidase procedure and purified by RP-HPLC at PerkinElmer (NEX207). The specific activity is about 2200 Ci/mmol. K_{D} determination is performed by homologous competition instead of saturation binding due to high propanol content in the ¹²⁵I-labelled glucagon material. The K_{D} for hGCGR binding is estimated to be 3.65 nM and is used to calculate Kᵢ values for all compounds tested.

The receptor binding assay is carried out using a Scintillation Proximity Assay (SPA) method (*see, e.g.,* Sun et al. (2005) Metab. Eng. 7:38-44) with WGA beads (PerkinElmer; Waltham, MA). Human glucagon and the compounds of Examples 1-4 are dissolved in DMSO at a concentration of 2 mM and stored frozen at -20°C.

The compounds of Examples 1-4 and human glucagon are serially diluted into DMSO. 10 µL of diluted test compounds or cold glucagon (non-specific binding (NSB)) at 1 µM final, are transferred into Corning^{®} 3604 (non-binding surface) clear bottom assay plates containing 45 µL assay binding buffer (25 mM HEPES, pH 7.4, 2.5 mM CaClz, 1 mM MgCl₂, 0.1 % (w/v) bacitracin, 0.003 % (w/v) Tween^{®} 20, and cOmplete^{™} protease inhibitors without EDTA). 90 µL membranes (1.5 µg/well), 50 µL ¹²⁵I-labelled glucagon (0.15 nM final concentration in reaction), and 50 µL of WGA beads (150 µg/well) are added. DMSO concentration does not exceed 4.2%. Plates are sealed, mixed on a plate shaker, and read with a MicroBeta^{™} scintillation counter (PerkinElmer) after 12 hr of settling time at room temperature.

Results are calculated as a percent of specific ¹²⁵I-labelled glucagon binding in the presence of the compounds of Examples 1-4. The absolute IC₅₀ concentration of the test compounds is derived by non-linear regression analysis of percent specific binding of ¹²⁵I-labelled glucagon vs. the concentration of sample added (5.1 × 10⁻¹¹ to 1.0 × 10⁻⁶ mol/L). The IC₅₀ concentration is converted to Kᵢ using the Cheng-Prusoff equation (Cheng & Prusoff (1973) Biochem. Pharmacol. 22:3099-3108).

2. Rat Glucagon Receptor Binding Assay: to determine whether the compounds bind to the rat glucagon receptor (rGCGR), a binding assay as essentially described in the human glucagon receptor binding assay is performed. Crude plasma membranes are prepared from 293-HEK cells in suspension culture transiently transfected with a cloned rGCGR (Svoboda et al. (1993) Biochem. Biophys.Res. Commun. 191:479-486; *see also,* GenBank Accession No. L04796.1 for the nucleotide sequence). Frozen cell pellets are lysed on ice in hypotonic buffer containing 25 mM Tris HCl, pH 7.5, 1 mM MgCl₂, 25 Units/ml DNAse I (Invitrogen; Carlsbad, CA) and cOmplete^{™} protease inhibitors without EDTA. The cell suspension is homogenized using a glass Potter-Elvehjem homogenizer fitted with a Teflon^{®} pestle for 20 strokes. The homogenate is centrifuged at 4°C at 1800 x g for 15 minutes. The supernatant is saved and stored on ice, and the pellets are re-suspended in homogenization buffer and re-homogenized. The homogenate is centrifuged at 1800 x g for 15 min. The second supernatant is combined with the first supernatant and centrifuged at 25000 x g for 30 min at 4°C. The resulting membrane pellet is re-suspended in homogenization buffer (without DNAse I) containing protease inhibitors at about 1 to 3 mg/mL, quick frozen in liquid nitrogen and stored as aliquots in a -80°C freezer until use.

Human glucagon (SEQ ID NO:1) is radioiodinated by ¹²⁵I-lactoperoxidase procedure and purified by reversed phase HPLC at PerkinElmer (NEX207). The specific activity is about 2200 Ci/mmol. K_{D} determination is performed by homologous competition instead of saturation binding due to high propanol content in the ¹²⁵I-labelled glucagon. The K_{D} for rMR binding is estimated to be 20.4 nM and is used to calculate Kᵢ values for all compounds tested.

The SPA receptor binding assay and calculation of the results are carried out as described in the hGCGR binding assay.

### Results

**Table 6. In Vitro Binding Affinity (Kᵢ) for the Compounds of Examples 1-4 and Comparator to rGCGR and hGCGR.**

| | Kᵢ, nM (SEM, n) | |
|---|---|---|
| | rGCGR | hGCGR |
| hGCG | 20.8 (1.3, 15) | 3.37 (0.31, 15) |
| Example 1 | 17.4 (1.5, 16) | 3.18 (0.34, 16) |
| Example 2 | 34.2 (2.9, 25) | 6.87 (0.62, 25) |
| Example 3 | 21.7 (2.0, 25) | 2.08 (0.32, 25) |
| Example 4 | 13.5 (1.4, 18) | 1.20 (0.21, 18) |

These data show that the compounds of Examples 1-4 bind to rGCGR or hGCGR with similar or increased affinity when compared to human glucagon and may activate that receptor, in turn triggering glucagon-dependent physiological responses.

### Example 10: Functional Activity of the Compounds of Examples 1-4

### Methods

Functional Activity Assay: radioligand competition binding assays are run to determine the equilibrium dissociation constant (K_{D}) for the compounds of Examples 1-4 and comparator compounds [human glucagon (SEQ ID NO:1), human GIP amide (SEQ ID NO:7) and human GLP-1 amide (SEQ ID NO:8)]. Such assay use SPA methods and membranes prepared from transfected HEK-293 cells overexpressing human GLP-1 receptor (hGLP-1R; *see,* GenBank Accession No. NM_002062)-, hGCGR-, and human GIP receptor (hGIP-R; *see,* GenBank Accession No. AAA84418.1)-expressing HEK-293 clonal cell lines. Each receptor over-expressing cell line is treated with test compound (20-point CRC, 2.75-fold Labcyte Echo direct dilution) in DMEM (Gibco Cat# 31053) supplemented with 1X GlutaMAXTM (Gibco Cat# 35050), 0.25% FBS (Fetal Bovine Serum, Gibco Cat# 26400), 0.05% fraction V BSA (Bovine Serum Albumin, Gibco Cat# 15260), 250 µM IBMX and 20 mM HEPES (Gibco Cat# 15630) in a 20 µl assay volume. After a sixtyminute incubation at room temperature, the resulting increase in intracellular cAMP is quantitatively determined using a cAMP Dynamic 2 HTRF Assay Kit (62AM4PEJ; CisBio; Codolet, FR). Briefly, cAMP levels within the cell are detected by adding a cAMP-d2 conjugate in cell lysis buffer (10 µl) followed by an anti-cAMP-Eu3+-Cryptate antibody, also in cell lysis buffer (10 µl). The resulting competitive assay is incubated for at least 60 min at room temperature, then detected using a PerkinElmer Envision^{®} instrument with excitation at 320 nm and emission at 665 nm and 620 nm. Envision units (emission at 665nm/620nm* 10,000) are inversely proportional to the amount of cAMP present and are converted to nM cAMP per well using a cAMP standard curve. The amount of cAMP generated (nM) in each well is converted to a percent of the maximal response observed with either human GLP-1(7-36)NH₂, human glucagon, or human GIP(1-42)NH₂. A relative EC₅₀ value and percent top (Eₘₐₓ) are derived by non-linear regression analysis using the percent maximal response vs. the concentration of peptide added, fitted to a four-parameter logistic equation.

For human GLP-2 receptor (hGLP-2R), a cloned human GLP-2-expressing cell line (20160624-BE03859-037) is used to assess hGLP-2R-stimulated cAMP functional response. Cells are stimulated with GLP-2 (SEQ ID NO:9) or the compounds of Examples 1-4, and the cAMP generated within the cell is quantitated using the CisBio cAMP Dynamic 2 HTRF Assay Kit (62AM4PEC). Briefly, cAMP levels within the cell are detected by binding to the cAMP-d2 capture antibody in the presence of cell lysis buffer. A second detection antibody provided in the kit, anti-cAMP Cryptate, is added to create a competitive sandwich assay. When the detection antibody complex formed there is an increase in the signal that is measured on a PerkinElmer Envision^{®} instrument.

The cryopreserved hGLP-2R-HEK-293 cells are quickly thawed at 37°C water bath and re-suspended in pre-warmed DMEM cell medium (Gibco 31053) supplemented with 0.5% defined FBS (Hyclone SH30070), GlutaMAX (Gibco 35050), and 20 mM HEPES (HyClone Cat# SH30237.01). Cells are then pelleted at 100 X g at room temperature for 5 min. The supernatant is removed, and the cell pellet is re-suspended in DMEM at 5 × 10⁴ cells/ml. Then, 50 µL of cells (2500 cell/well) are seeded to 96-well Half Area Black plates (Costar 3875) for overnight incubation at 37°C incubator. Test samples are prepared as 2 mM stocks in DMSO and frozen at -20°C until needed. On the day of the treatment, GLP-2, buffer controls and the compounds of Examples 1-4, are serially diluted into DMSO followed by a step-down dilution into Compound Dilution Media (Assay Media (DMEM, Gibco 31053P with 0.1% fatty acid-free bovine serum albumin, BSA, 7.5%, (Gibco 15620); 20 mM HEPES, pH 7.4, and 2 mM GlutaMAX) that contains 500 µmοl/L IBMX). Before the reaction, cells are washed with 100 µL/well of Assay Media and replaced with 20 µL/Assay Media, then the reaction is followed by addition of 20 µL of either 2x-concentrated GLP-2, buffer controls or the compounds of Examples 1-4 in Compound Dilution Media. Final DMSO concentration does not exceed 0.5%, and final IBMX concentration is 250 µM. After 1 hr incubation at room temperature, the reaction is stopped by addition of 20 µL of the cAMP-d2-capture antibody (CisBio) diluted into the CisBio lysis buffer and gently mixed in a TitraMax^{™} shaker (Heidolph Instruments GmbH & Co KG; Schwabach, DE) for 1 min, then 20 µL of the detection antibody, anti-cAMP Cryptate (CisBio) added and mixed at 600 rpm for 1 min, followed by gently shaking at 300 rpm at room temperature. The lysed cell and antibody mixtures are read after 1 hr using the PerkinElmer Envision^{®} instrument. Envision units were converted to pmol/L cAMP/well using the cAMP standard curve. Picomoles of cAMP generated in each well is converted to a percent of the maximal response observed with the GLP-2 control. A relative EC₅₀ value is derived by non-linear regression analysis using the percent maximal response vs. the concentration of peptide added, fitted to a four-parameter logistic equation (Genedata Screener 13).

The cloned rat glucagon expressing cell line (rGR C#18, BE03581-029-BE03754-065) is used for rat glucagon receptor stimulated cAMP functional assay. Cells are stimulated with glucagon, or Test samples, and the cAMP generated within the cell is quantitated using the CisBio cAMP Dynamic 2 HTRF Assay Kit (62AM4PEC). Briefly, cAMP levels within the cell are detected by binding to the cAMP-d2 capture antibody in the presence of cell lysis buffer. A second detection antibody provided in the kit, anti-cAMP Cryptate, is added to create a competitive sandwich assay. When the detection antibody complex formed there is an increase in the signal that is measured on a PerkinElmer Envision^{®} instrument.

On the day of experiment, cryo-preserved ratGlucagonReceptor-HEK293 cells are quickly thawed at 37°C water bath and resuspended in pre-warmed Cell Media DMEM (Gibco 31053) supplemented with 0.5% defined FBS (Hyclone SH30070), GlutaMAX (Gibco 35050), and 20 mM HEPES (HyClone Cat# SH30237.01). Cells are then pelleted at 100 X g at room temperature for 5 minutes. The supernatant is removed and the cell pellet is resuspended in Cell Media. Test samples are prepared as 2 mM stocks in DMSO and frozen at -20°C until needed. Glucagon, buffer controls and test compounds, are serially diluted into DMSO followed by a step-down dilution into Compound Dilution Media (Assay Media (DMEM, Gibco 31053P with 0.1% fatty acid-free bovine serum albumin, BSA, 7.5%, (Gibco 15620); 20 mM HEPES, pH 7.4, and 2 mM GlutaMAX) that contains 500 µmol/L IBMX). The reaction is performed in 40 µL, by adding 20 µL of cells (2500 cell/well) or cAMP standard curve samples to 96 Well plate Half Area Black plates (Costar 3694), followed by addition of 20 µL of either 2X concentrated glucagon, buffer controls or test compounds in Compound Dilution Media. Final DMSO concentration does not exceed 0.5%, and final IBMX concentration is 250 µM. After 1 hour incubation at room temperature, the reaction is stopped by addition of 20 µL of the cAMP-d2-capture antibody (CisBio) diluted into the CisBio lysis buffer and gently mixed in TITRAMAX shaker for 1minute, then 20 µL of the detection antibody [anti-cAMP Cryptate (CisBio)] is added and mixed at 600 rpm for 1 minute on a plate shaker followed by gently shaking at 300rpm at room temperature. The cell lysate and antibody mixtures are read after 1 hour using the Perkin-Elmer Envision^{®}. Envision^{®} units were converted to pmol/L cAMP/well using the cAMP standard curve. Picomoles of cAMP generated in each well is converted to a percent of the maximal response observed with the glucagon control. A relative EC₅₀ value is derived by non-linear regression analysis using the percent maximal response vs. the concentration of peptide added, fitted to a four-parameter logistic equation (Genedata Screener 13).

### Results

**Table 7: Functional cAMP Potency (EC₅₀) for Compounds of Examples 1-4 and Comparators at hGCGR, hGLP-1R and GIPR.**

| | cAMP EC₅₀ (nM; SEM, n) | | |
|---|---|---|---|
| | hGCGR | hGIPR | hGLP-1R |
| hGCG | 0.00908 (0.00080, 79) | | 10.9 (6.05, 236) |
| hGIP amide | | 0.196 (0.016, 69) | |
| hGLP-1 amide | | | 0.0870 (0.0095, 66) |
| Example 1 | 0.0143 (0.0011, 29) | >9950 (n=30) | 181 (13, 26) |
| Example 2 | 0.0265 (0.0023, 32) | >9950 (n = 25) | 250 (25, 25) |
| Example 3 | 0.0108 (0.0013, 27) | >9950 (n=23) | 1300 (140, 25) |
| Example 4 | 0.00888 (0.00069, 26) | >9950 (n=28) | 1270 (140, 19) |

These data show that in the presence of FBS and BSA, the compounds of Examples 1-4 have agonist activities at hGCGR equal or greater than human glucagon. In addition, these data show that Examples 1-4 have weaker agonist activity at hGLP-1R than human glucagon, which indicates that Examples 1-4 have greater degree of selectivity than human glucagon. In addition, the compounds of Examples 1-4 do not appreciably bind and activate hGIPR. As such, Examples 1-4 are predicted to initiate glucagon receptor mediated physiological responses but not to initiate GLP-1R and GIPR-mediated physiological responses.

**Table 8: Efficacy (Eₘₐₓ) for the Compounds of Examples 1-4 and Comparators at hGCGR, hGLP-1R and hGIPR.**

| | Eₘₐₓ (%^{a} ± SEM) | | |
|---|---|---|---|
| | hGCGR | hGIPR | hGLP-1R |
| hGCG | 107 ± 1 | | 104± 1 |
| hGIP amide | | 106 ± 2 | |
| hGLP-1 amide | | | 104 ± 2 |
| Example 1 | 103 ± 2 | < 5% (n=30) at 9.95 µM | 105 ± 2 |
| Example 2 | 106 ± 1 | < 5% (n=25) at 9.95 µM | 109 ± 4 |
| Example 3 | 106 ± 2 | < 5% (n=23) at 9.95 µM | 109 ± 5 |
| Example 4 | 98 ± 2 | < 5% (n=28) at 9.95 µM | 100 ± 5 |

| | | | |
|---|---|---|---|
| ^{a} Eₘₐₓ, % = the Arithmetic Mean ± SE for the percent of response to 10 nM GLP-1(7-36)NH₂, 1 nM Glucagon or 10 nM GIP(1-42)NH₂. | | | |

These data show that with regard to efficacy Examples 1-4 of the present invention behave very similar to human glucagon as being full efficacious in the GcgR and GLP-1R cAMP agonist assays.

**Table 9: Functional cAMP Potency (EC₅₀) at ratGCGR and hGLP-2R for the Compounds of Examples 1-4 and Comparators.**

| | cAMP EC₅₀ (nM; SEM, n) | |
|---|---|---|
| | rGCGR | hGLP-2R |
| hGCG | 0.111 (0.012, 16) | |
| hGLP-2 | | 0.016 (0.001, 5) |
| Example 1 | 0.135 (0.033, 5) | ND |
| Example 2 | 0.204 (0.022, 12) | ND |
| Example 3 | 0.188 (0.029, 8) | ND |
| Example 4 | 0.083 (0.007, 5) | ND |

These data (left column) show that Examples 1-4 and native glucagon functionally activate rat GCGR with similar potency and can thereby initiate glucagon receptor-mediated physiological responses in rats. These data (right column) show that the compounds of Examples 1-4 do not stimulate cAMP at hGLP-2R at the highest concentration of 2500 nM and therefore can not functionally activate hGLP-2R. As such, they are predicted not to initiate GLP-2R-mediated physiological responses.

### Example 11: Immunogenicity of the Compounds of Examples 1-4

### Methods

1. CD4+ T Cell Assay: to assess the propensity for a clinical immunogenic response to the Gcompounds of Examples 1-4, CD8+ T cell-depleted peripheral blood mononuclear cells (PBMC's) are prepared and labeled with carboxyfluorescein diacetate succinimidyl ester (CFSE; Invitrogen) from a cohort of 10 healthy donors with diverse human leukocyte antigen (HLA) class II haplotypes. Each donor is tested in triplicate with 2.0 mL media control, keyhole limpet haemocyanin (KLH; 0.33µM), and the compounds of Examples 1-4 (10 µM). Cultures are incubated for 7 days at 37°C with 5% CO₂. On day 7, samples are analyzed by flow cytometry using a BD LSR II Fortessa (Becton Dickinson; Franklin Lakes, NJ), equipped with a high throughput sampler (HTS). Data is analyzed using FlowJo^{®} Software (FlowJo, LLC/TreeStar; Ashland, OR).

The CD4+ t cell assay therefore is used to compare the compounds of Examples 1-4 for a potential to induce an immune response *in vivo* according to methods known in the art *(see, e.g.,* Jones et al. (2004) J. Interferon Cytokine Res. 24:560-572; and Jones et al. (2005) J. Thromb. Haemost. 3:991-1000), where an assessment of clinically tested monoclonal antibodies and peptides shows some degree of correlation between T cell proliferation observed *in vitro* and immunogenicity in the clinic. Protein therapeutics that induce less than 30% positive response in the CD4+ T cell proliferation assay are associated with a low risk of immunogenicity.

### Results

**Table 10: CD4+ T Cell Responses for the Compounds of Examples 1-4 and Positive Control.**

| | % Donor Response (n=10) | Median Response Strength in positive donors (CDI) |
|---|---|---|
| KLH | 100% | 131 (n=10) |
| Example 1 | 0% | NA (n=0) |
| Example 2 | 0% | NA (n=0) |
| Example 3 | 10% | 6.5 (n=1) |
| Example 4 | 20% | 6.42 (n=2) |

| | | |
|---|---|---|
| Cell Division Index ("CDI"): proportion of divided CD4+ T cells to the total number of CD4+ T cells in stimulated versus unstimulated samples. | | |

These data show that the frequency of positive CD4+ T cell response (CDI>2.5) was low for the compounds of Examples 1-4, and the magnitude of the response in the few positive donors was low (CDI<7), indicating a low risk of immunogenicity.

### IN VIVO STUDIES

### Example 12: Pharmacokinetics of the Compounds of Examples 1-4

### Methods

1. Bioanalytics: plasma concentration of the compounds of Examples 1-4 are determined by a qualified Liquid Chromatography Mass Spectrometry (LC/MS) method at Q Squared Solutions BioSciences LLC (Ithaca, NY) or at Algorithme Pharma (Laval, Quebec, CA). The compounds and an analog as an internal standard are extracted from 100% species-specific by antibody or protein precipitations. The intact mass of the compounds are detected by a Q Exactive^{®} Orbitrap^{®} Mass Spectrometer (Thermo Fisher Scientific; Waltham, MA).
2. Pig PK Studies: In one study (Table 11), male Yucatan Miniature Swine (MS) are administered a single subcutaneous dose (30 µg/kg) of Example 2 compound in histidine with propylene glycol buffer (pH 6) (H6PG) or are administered recombinant, human glucagon (rGlucagon, E-kit) at a volume of 0.010 mL/kg. Blood is collected at pre-dose, 2, 5, 10, 15, 20, 25, 30, 45, 60, 75, 90, 120, 150, 180 and 240 minutes post-dose, and plasma is then obtained for pharmacokinetic characterization.

In another study (Table 12), male Yucatan MS are administered a single subcutaneous dose (30 µg/kg) of Example 2 compound in H6PG or are administered rGlucagon at a volume of 0.010 mL/kg. Blood is collected at pre-dose, 2, 5, 10, 15, 20, 25, 30, 45, 60, 75, 90, 120, 150, 180 and 240 minutes post-dose, and plasma is then obtained for pharmacokinetic characterization.

In another study (Table 13), male Yucatan MS are administered a single subcutaneous dose (10 or 30 µg/kg) of Example 2 compound in H6PG or are administered rGlucagon at a volume of 0.010 mL/kg. Blood is collected at pre-dose, 2, 5, 10, 15, 20, 25, 30, 45, 60, 75, 90, 120, 150, 180 and 240 minutes post-dose, and plasma is obtained for pharmacokinetic characterization.

In another study (Table 14), male Yucatan MS are administered a single subcutaneous dose (10 or 30 µg/kg) of Example 3 compound in H6PG at a volume of 0.010 mL/kg. Blood is collected at pre-dose, 2, 5, 10, 15, 20, 25, 30, 45, 60, 75, 90, 120, 150, 180 and 240 minutes post-dose, and plasma is obtained for pharmacokinetic characterization.

In another study (Table 15), male Yucatan MS are administered a single subcutaneous dose (10 or 30 µg/kg) of Example 1, Example 3 and Example 4 compounds in H6PG at a volume of 0.010 mL/kg. Blood is collected at pre-dose, 2, 5, 10, 15, 20, 25, 30, 45, 60, 75, 90, 120 and 150 minutes post-dose, and plasma is obtained for pharmacokinetic characterization.

In another study (Table 16), male Yucatan MS are administered a single subcutaneous dose (10 or 30 µg/kg) of Example 1, Example 3 and Example 4 compounds in H6PG at a volume of 0.010 mL/kg. Blood is collected at pre-dose, 2, 5, 10, 15, 20, 25, 30, 45, 60, 75, 90, 120 and 150 minutes postdose, and plasma is obtained for pharmacokinetic characterization.

### Results

**Table 11: Individual and Mean Plasma PK Parameters Following a Single 30 µg/kg Subcutaneous Dose to Male Yucatan MS.**

| Compound (dose) | Animal | T_{1/2} (min) | Tₘₐₓ (min) | Cₘₐₓ (ng/mL) | AUC_{0-inf} (min*ng/mL) | CL/F (mL/min/kg) |
|---|---|---|---|---|---|---|
| Example 2 (30 µg/kg) | 1 | 20.5 | 15 | 24.9 | 590 | 50.83 |
| | 2 | 44.4 | 20 | 13.5 | 712 | 42.13 |
| | 3 | 71.4 | 30 | 17.2 | 732 | 40.99 |
| | 4 | 50.1 | 15 | 15.6 | 875 | 34.27 |
| | 5 | 36.7 | 25 | 10.2 | 555 | 54.01 |
| | 6 | 36.4 | 60 | 5.3 | 428 | 70.14 |
| | Mean | 43.2 | 28 | 14.4 | 649 | 48.73 |
| | SD | 17.0 | 17 | 6.6 | 157 | 12.68 |
| rGCG (30 µg/kg) | 7 | 26.9 | 30 | 5.1 | 423 | 70.89 |
| | 8 | 70.0 | 20 | 7.7 | 920 | 32.60 |
| | 9 | 225.5 | 5 | 11.4 | 1786 | 16.80 |
| | Mean | 107.4 | 18 | 8.1 | 1043 | 40.09 |
| | SD | 104.5 | 13 | 3.2 | 690 | 27.81 |

| | | | | | | |
|---|---|---|---|---|---|---|
| Abbreviations: T_{1/2} = half-life, Tₘₐₓ = time to maximum concentration, Cₘₐₓ = maximum observed plasma concentration, AUC_{0-inf} = area under the curve from time 0 hours to infinity, CL/F = clearance/bioavailability, rGCG = recombinant, human glucagon; SD = standard deviation. | | | | | | |

**Table 12: Individual and Mean Plasma PK Parameters Following a Single 30 µg/kg Subcutaneous Dose to Male Yucatan MS.**

| Compound (dose) | Animal | T_{1/2} (min) | Tₘₐₓ (min) | Cₘₐₓ (ng/mL) | AUC_{0-inf} (min*ng/mL) | CL/F (mL/min/kg) |
|---|---|---|---|---|---|---|
| Example 2 (30 µg/kg) | 8 | 50.4 | 25 | 6.3 | 411 | 72.98 |
| | 10 | 19.0 | 30 | 17.4 | 766 | 39.18 |
| | 11 | 25.8 | 20 | 9.8 | 548 | 54.75 |
| | 12 | 51.9 | 20 | 31.0 | 775 | 38.72 |
| | 9 | 12.4 | 15 | 10.1 | 610 | 49.22 |
| | Mean | 31.9 | 22 | 14.9 | 622 | 50.97 |
| | SD | 18.2 | 6 | 9.9 | 153 | 14.06 |
| rGCG (30 µg/kg) | 13 | 81.3 | 25 | 9.3 | 864 | 34.73 |
| | 14 | 75.5 | 15 | 7.9 | 548 | 54.75 |
| | 15 | 61.8 | 25 | 20.6 | 1147 | 26.16 |
| | 16 | 35.6 | 25 | 7.3 | 594 | 50.53 |
| | 17 | 60.3 | 30 | 9.4 | 492 | 61.00 |
| | Mean | 62.9 | 24 | 10.9 | 729 | 45.43 |
| | SD | 17.7 | 5 | 5.5 | 274 | 14.50 |

| | | | | | | |
|---|---|---|---|---|---|---|
| Abbreviations: T_{1/2} = half-life, Tₘₐₓ = time to maximum concentration, Cₘₐₓ = maximum observed plasma concentration, AUC_{0-inf} = area under the curve from time 0 hours to infinity, CL/F = clearance/bioavailability, rGCG = recombinant, human glucagon; SD = standard deviation. | | | | | | |

**Table 13: Individual and Mean Plasma PK Parameters Following a Single 10 or 30 µg/kg Subcutaneous Dose to Male Yucatan MS.**

| Compound (dose) | Animal | T_{1/2} (min) | Tₘₐₓ (min) | Cₘₐₓ (ng/mL) | AUC_{0-inf} (min*ng/mL) | CL/F (mL/min/kg) |
|---|---|---|---|---|---|---|
| Example 2 (10 µg/kg) | 8 | 32.2 | 10 | 1.6 | 87 | 114.90 |
| | 10 | 18.5 | 15 | 3.5 | 115 | 86.95 |
| | 11 | 14.9 | 25 | 2.3 | 92 | 109.05 |
| | 12 | 28.5 | 45 | 1.4 | 95 | 105.64 |
| | 9 | 33.5 | 25 | 1.3 | 87 | 115.23 |
| | Mean | 25.5 | 24 | 2.0 | 95 | 106.35 |
| | SD | 8.4 | 13 | 0.9 | 12 | 11.58 |
| rGCG (10 µg/kg) | 13 | 30.4 | 45 | 1.8 | 125 | 80.05 |
| | 14 | 22.3 | 15 | 2.6 | 87 | 115.47 |
| | 15 | NR | 90 | 1.4 | NR | NR |
| | 16 | 14.3 | 10 | 2.6 | 97 | 103.31 |
| | 17 | 18.4 | 5 | 3.4 | 65 | 154.58 |
| | Mean | 21.3 | 33 | 2.4 | 93 | 113.35 |
| | SD | 6.8 | 35 | 0.8 | 25 | 31.17 |
| rGCG (30 µg/kg) | 5 | 51.3 | 25 | 3.9 | 359 | 83.55 |
| | 2 | 31.5 | 25 | 6.3 | 399 | 75.25 |
| | 4 | 35.5 | 10 | 16.4 | 725 | 41.40 |
| | 3 | 41.5 | 15 | 5.1 | 413 | 72.61 |
| | 6 | 70.6 | 5 | 5.1 | 680 | 44.10 |
| | 1 | 47.7 | 5 | 5.3 | 385 | 78.02 |
| | Mean | 46.3 | 14 | 7.0 | 493 | 65.82 |
| | SD | 14.0 | 9 | 4.7 | 164 | 18.26 |

| | | | | | | |
|---|---|---|---|---|---|---|
| Abbreviations: T_{1/2} = half-life, Tₘₐₓ = time to maximum concentration, Cₘₐₓ = maximum observed plasma concentration, AUC_{0-inf} = area under the curve from time 0 hours to infinity, CL/F = clearance/bioavailability, rGCG = recombinant, human glucagon; SD = standard deviation. | | | | | | |

**Table 14: Individual and Mean Plasma PK Parameters Following a Single 10 or 30 µg/kg Subcutaneous Dose to Male Yucatan MS.**

| Compound (dose) | Animal | T_{1/2} | Tₘₐₓ | Cₘₐₓ | AUC_{0-inf} | CL/F |
|---|---|---|---|---|---|---|
| | | (min) | (min) | (ng/mL) | (min^{∗}ng/mL) | (mL/min/kg) |
| Example 3 (10 µg/kg) | 8 | NR | 25 | 2.7 | NR | NR |
| | 10 | 26.8 | 15 | 1.8 | 79 | 127.01 |
| | 11 | NR | 30 | 1.5 | NR | NR |
| | 12 | 23.5 | 25 | 2.5 | 129 | 77.85 |
| | 9 | 21.4 | 45 | 2.9 | 164 | 61.03 |
| | Mean | 24.0 | 28 | 2.3 | 124 | 88.63 |
| | SD | 2.7 | 11 | 0.6 | 43 | 34.29 |
| Example 3 (30 µg/kg) | 5 | 55.2 | 45 | 5.0 | 355 | 84.50 |
| | 2 | 17.8 | 25 | 7.3 | 367 | 81.71 |
| | 4 | 39.2 | 15 | 7.6 | 512 | 58.57 |
| | 3 | 45.9 | 30 | 6.0 | 351 | 85.47 |
| | 1 | 29.8 | 15 | 3.9 | 195 | 153.71 |
| | Mean | 37.6 | 26 | 6.0 | 356 | 92.79 |
| | SD | 14.5 | 13 | 1.6 | 112 | 35.80 |

| | | | | | | |
|---|---|---|---|---|---|---|
| Abbreviations: T_{1/2} = half-life, Tₘₐₓ = time to maximum concentration, Cₘₐₓ = maximum observed plasma concentration, AUC_{0-inf} = area under the curve from time 0 hours to infinity, CL/F = clearance/bioavailability, NR - not reported, SD = standard deviation. | | | | | | |

**Table 15: Individual and Mean Plasma PK Parameters Following a Single 10 or 30 µg/kg Subcutaneous Dose to Male Yucatan MS.**

| Compound (dose) | Animal | T_{1/2} (min) | Tₘₐₓ (min) | Cₘₐₓ (ng/mL) | AUC_{0-inf} (min*ng/mL) | CL/F (mL/min/kg) |
|---|---|---|---|---|---|---|
| Example 1 (30 µg/kg) | 8 | 84.4 | 25 | 8.0 | 611 | 49.06 |
| | 2 | 26.3 | 15 | 10.5 | 365 | 82.30 |
| | 13 | 25.9 | 45 | 3.2 | 251 | 119.57 |
| | 1 | 39.1 | 15 | 5.4 | 294 | 101.96 |
| | 12 | 33.5 | 45 | 6.6 | 529 | 56.76 |
| | 9 | 13.8 | 30 | 15.7 | 952 | 31.52 |
| | 17 | 25.3 | 25 | 3.5 | 235 | 127.80 |
| | Mean | 35.5 | 29 | 7.6 | 462 | 81.28 |
| | SD | 22.9 | 12 | 4.4 | 258 | 36.91 |
| Example 4 (30 µg/kg) | 18 | 53.0 | 60 | 2.9 | 255 | 117.75 |
| | 10 | 37.2 | 25 | 7.3 | 373 | 80.41 |
| | 15 | 61.8 | 45 | 10.1 | 1014 | 29.60 |
| | 3 | NR | 90 | 3.7 | NR | NR |
| | 16 | 19.8 | 25 | 5.3 | 253 | 118.49 |
| | Mean | 43.0 | 49 | 5.9 | 474 | 86.56 |
| | SD | 18.5 | 27 | 2.9 | 364 | 41.94 |
| Example 3 (10 µg/kg) | 5 | NR | 25 | 1.6 | NR | NR |
| | 14 | NR | 30 | 1.9 | NR | NR |
| | Mean | NR | 28 | 1.8 | NR | NR |

| | | | | | | |
|---|---|---|---|---|---|---|
| Abbreviations: T_{1/2} = half-life, Tₘₐₓ = time to maximum concentration, Cₘₐₓ = maximum observed plasma concentration, AUC_{0-inf} = area under the curve from time 0 hours to infinity, CL/F = clearance/bioavailability, NR - not reported, SD = standard deviation. | | | | | | |

**Table 16: Individual and Mean Plasma PK Parameters Following a Single 10 or 30 µg/kg Subcutaneous Dose to Male Yucatan MS.**

| Compound (dose) | Animal | T_{1/2} (min) | Tₘₐₓ (min) | Cₘₐₓ (ng/mL) | AUC_{0-inf} (min*ng/mL) | CL/F (mL/min/kg) |
|---|---|---|---|---|---|---|
| Example 1 (10 µg/kg) | 8 | NR | 20 | 1.7 | NR | NR |
| | 2 | NR | 20 | 1.8 | NR | NR |
| | 13 | NR | NR | NR | NR | NR |
| | 1 | NR | 25 | 2.6 | NR | NR |
| | 9 | NR | 25 | 3.2 | NR | NR |
| | 17 | NR | 25 | 1.0 | NR | NR |
| | Mean | NR | 23 | 2.1 | NR | NR |
| | SD | NR | 3 | 0.9 | NR | NR |
| Example 4 (10 µg/kg) | 5 | NR | 25 | 2.0 | NR | NR |
| | 10 | NR | 30 | 4.7 | NR | NR |
| | 15 | NR | 45 | 1.6 | NR | NR |
| | 3 | NR | 30 | 1.8 | NR | NR |
| | 16 | NR | 45 | 2.6 | NR | NR |
| | Mean | NR | 35 | 2.5 | NR | NR |
| | SD | NR | 9 | 1.2 | NR | NR |
| Example 3 (30 µg/kg) | 18 | 136.9 | 25 | 1.9 | 338 | 88.83 |
| | 14 | 26.6 | 45 | 6.3 | 430 | 69.84 |
| | Mean | 81.8 | 35 | 4.1 | 384 | 79.34 |

| | | | | | | |
|---|---|---|---|---|---|---|
| Abbreviations: T_{1/2} = half-life, Tₘₐₓ = time to maximum concentration, Cₘₐₓ = maximum observed plasma concentration, AUC_{0-inf} = area under the curve from time 0 hours to infinity, CL/F = clearance/bioavailability, NR = not reported, SD = standard deviation. | | | | | | |

These data show that pharmacokinetic parameters for Examples 1-4 and human recombinant glucagon are very similar in a pig model after single administration at clinical relevant dose levels. This data predicts that pharmacokinetic profiles on Examples 1-4 and human recombinant glucagon might be similar in humans as well.

### Example 13: Effect of the Compounds of Examples 1-4 on Blood Glucose in a Rat Model

### Methods

1. Animal Protocol: four-month-old, male, Sprague-Dawley rats (Envigo; Indianapolis, IN) are used. Animals are individually housed in a temperature-controlled (24°C) facility with a 12-hour light/dark cycle, and have free access to food and water. After a one-week acclimation to the facility, rats are randomized to treatment groups (n= 4/group). Test compound is formulated in a buffer (10 mM histidine, 19 mg/mL PG, pH 6). On the morning of testing, food is removed at 06:00 a.m. Two hours after food is removed, the test compound is given subcutaneously at 0, 3, 10, 30 or 100 µg/kg doses. Blood glucose is measured at time 0, 3, 6, 9, 15, 20, 30, 45 and 60 min after administration via an Accu-Check^{®} glucometer (Roche Diabetes Care, Inc.; Indianapolis, IN).
   Insulin is measured at 3, 9, 15, 20, 30 and 60 min after administration via ELISA (MSD; Rockville, MD).
2. Statistics: results are expressed as mean ± standard error mean (SEM) of four rats per group. Statistical differences are assessed as *p<0.05 compared to corresponding time point of vehicle group by two-way ANOVA with Dunnett's multiple comparison test.

### Results

**Table 17: Blood Glucose Levels After Administering Example 1.**

| | Blood glucose levels (mg/dL) after dosing | | | | |
|---|---|---|---|---|---|
| Time (min) | 0 | 3 µg/kg | 10 µg/kg | 30 µg/kg | 100 µg/kg |
| 0 | 120.6±6.6 | 118.4±2.0 | 118.8±3.4 | 122.0±6.4 | 116.8±1.4 |
| 3 | 121.8±9.5 | 115.4±4.5 | 120.9±7.2 | 137.3±15.9 | 115.6±2.6 |
| 6 | 141.5±10.7 | 147.5±4.5 | 152.0±18.1 | 172.5±11.6 | 166.9±8.9 |
| 9 | 149.8±9.5 | 154.9±7.9 | 162.9±13.9 | 195.9±7.1^{∗} | 178.4±6.1 |
| 15 | 142.0±4.9 | 161.0±4.6 | 187.0±13.5^{∗} | 204.9±8.9* | 193.6±5.9* |
| 20 | 144.9±8.4 | 160.0±7.2 | 179.5±4.4* | 210.3±8.3 * | 189.8±5.9* |
| 30 | 133.8±8.9 | 146.0±7.2 | 160.1±3.9 | 172.9±10.6^{∗} | 174.0±6.2* |
| 45 | 129.1±5.6 | 133.8±6.4 | 140.0±4.5 | 147.0±7.0 | 146.4±8.1 |
| 60 | 125.8±6.3 | 125.1±2.6 | 130.4±3.3 | 134.5±2.9 | 131.1±4.8 |

**Table 18: Blood Glucose Levels After Administering Example 2.**

| | Blood glucose levels (mg/dL) after dosing | | | | |
|---|---|---|---|---|---|
| Time (min) | 0 | 3 µg/kg | 10 µg/kg | 30 µg/kg | 100 µg/kg |
| 0 | 112.0±4.7 | 109.9±2.7 | 110.3±2.5 | 113.4±1.7 | 105.1±1.4 |
| 3 | 119.5±5.2 | 117.0±4.1 | 121.8±6.9 | 127.8±3.2 | 123.8±8.2 |
| 6 | 125.6±5.8 | 124.1±11.4 | 135.4±11.3 | 152.9±10.3^{∗} | 144.9±9.9 |
| 9 | 136.3±6.7 | 139.1±6.8 | 160.1±9.2* | 195.8±7.8* | 177.9±5.4* |
| 15 | 151.3±4.0 | 160.6±9.6 | 194.3±11.9* | 209.5±7.8* | 192.6±4.9* |
| 20 | 142.4±5.9 | 139.5±7.3 | 168.4±5.5* | 181.0±6.1^{∗} | 172.6±7.3 * |
| 30 | 144.5±7.4 | 131.1±6.1 | 145.9±6.2 | 149.0±4.9 | 180.3±3.6* |
| 45 | 137.1±6.6 | 120.4±5.2 | 131.8±5.2 | 131.0±0.6 | 136.9±2.5 |
| 60 | 126.0±2.7 | 121.6±5.4 | 129.5±6.0 | 124.5±2.1 | 120.1±1.0 |

**Table 19: Blood Glucose Levels After Administering Example 3.**

| | Blood glucose levels (mg/dL) after dosing | | | | |
|---|---|---|---|---|---|
| Time (min) | 0 | 3 µg/kg | 10 µg/kg | 30 µg/kg | 100 µg/kg |
| 0 | 109.4±1.1 | 111.1±1.2 | 115.0±0.7 | 108.8±1.7 | 108.5±3.0 |
| 3 | 116.5±2.7 | 129.6±9.1 | 137.3±6.6 | 115.9±1.7 | 111.8±3.2 |
| 6 | 127.9±7.2 | 141.0±8.0 | 161.0±6.7* | 138.5±6.6 | 148.8±15.1 |
| 9 | 136.3±6.9 | 151.8±10.8 | 183.3±7.6* | 170.5±14.0* | 158.9±12.4 |
| 15 | 136.8±8.6 | 173.6±12.8* | 196.6±4.8* | 181.4±4.0* | 191.1±16.1^{∗} |
| 20 | 138.1±9.4 | 170.3±10.2* | 185.6±12.1^{∗} | 172.8±4.2* | 194.6±10.1^{∗} |
| 30 | 131.5±7.2 | 157.6±9.2 | 145.6±5.3 | 144.8±6.6 | 186.8±5.7* |
| 45 | 121.0±5.9 | 139.5±7.8 | 126.6±2.9 | 122.8±2.9 | 136.0±6.6 |
| 60 | 112.6±4.9 | 125.8±6.2 | 121.5±2.8 | 114.0±1.2 | 123.9±1.7 |

**Table 20: Blood Glucose Levels After Administering Example 4.**

| | Blood glucose levels (mg/dL) after dosing | | | | |
|---|---|---|---|---|---|
| Time (min) | 0 | 1 µg/kg | 3 µg/kg | 10 µg/kg | 30 µg/kg |
| 0 | 111.0±3.7 | 113.6±2.6 | 112.6±1.1 | 116.1±2.3 | 107.3±1.9 |
| 3 | 113.5±3.7 | 118.4±2.9 | 114.6±1.5 | 135.0±10.5* | 114.8±2.8 |
| 6 | 115.5±5.6 | 121.3±4.7 | 119.5±3.0 | 163.4±5.0* | 130.3±6.9 |
| 9 | 130.1±5.5 | 145.8±7.5 | 147.4±7.3 | 189.9±9.6* | 157.6±7.6* |
| 15 | 129.4±7.7 | 148.8±3.8 | 169.6±9.0* | 217.5±4.2* | 173.8±9.6* |
| 20 | 127.8±6.7 | 142.0±2.2 | 168.4±6.3 * | 208.4±8.6* | 173.8±9.4* |
| 30 | 128.4±2.5 | 135.6±2.5 | 148.4±4.4 | 180.4±12.9* | 151.9±10.5* |
| 45 | 126.6±4.2 | 132.0±2.8 | 136.0±3.4 | 145.3±6.5 | 130.3±5.6 |
| 60 | 122.3±3.2 | 125.1±4.5 | 127.4±2.5 | 132.6±4.1 | 117.3±3.6 |

These data show that the compounds of Examples 1-4 dose dependently increase blood glucose in a rat model. Profile of the glucose excursion is very similar to a dose of 10 ug/kg of native recombinant glucagon formulated in emergency-kit buffer solution (data not shown).

### SEQUENCES

The following nucleic and amino acid sequences are referred to in this disclosure and are provided below for reference.
SEQ ID NO: 1 - Human glucagon/Recombinant human glucagon
   HSQGTFTSDYSKYLDSRRAQDFVQWLMNT
SEQ ID NO:2 - Glucagon analog agonist
   HSX₁GTFTSDYSKYLD(Aib)RRAQX₂FVK(4-Pal)LLST_{-OH}, where
   X₁ is Q or Dab(Ac); and
   X₂ is Q or A
SEQ ID NO:3 - Glucagon analog agonist
   HSQGTFTSDYSKYLD(Aib)RRAQQFVK(4-Pal)LLST_{-OH}
SEQ ID NO:4 - Glucagon analog agonist
   HSQGTFTSDYSKYLD(Aib)RRAQAFVK(4-Pal)LLST_{-OH}
SEQ ID NO:5 - Glucagon analog agonist
   HS[Dab(Ac)]GTFTSDYSKYLD(Aib)RRAQAFVK(4-Pal)LLST-_{OH}
SEQ ID NO:6 - Glucagon analog agonist
   HS[Dab(Ac)]GTFTSDYSKYLD(Aib)RRAQQFVK(4-Pal)LLST-_{OH}
SEQ ID NO:7 - Human GIP (1-42) amide
   YAEGTFISDYSIAMDKIHQQDFVNWLLAQKGKKNDWKHNITQ-_{NH2}
SEQ ID NO:8 - Human GLP-1 (7-36) amide
   HAEGTFTSDVSSYLEGQAAKEFIAWLVKGR-_{NH2}
SEQ ID NO:9 - GLP-2 (1-34) acid
   HADGSF SDEMNTILDNLAARDFINWLIQTKITDR-_{OH}

## Claims

1. A compound comprising an amino acid sequence of
HSXiGTFTSDYSKYLD(Aib) RRAQX₂FVK(4-Pal)LLST, wherein X₁ is Q or Dab(Ac) and X₂ is Q or A (SEQ ID NO:2),

2. The compound of claim 1, wherein X₁ is Q and X₂ is Q (SEQ ID NO:3).

3. The compound of claim 1, wherein X₁ is Q and X₂ is A (SEQ ID NO:4).

4. The compound of claim 1, wherein X₁ is Dab(Ac) and X₂ is A (SEQ ID NO:5).

5. The compound of claim 1, wherein X₁ is Dab(Ac) and X₂ is Q (SEQ ID NO:6).

6. A compound of claim 2 which is HSQGTFTSDYSKYLD(Aib)RRAQQFVK(4-Pal)LLST-OH (SEQ ID NO:3).

7. A compound of claim 3 which is HSQGTFTSDYSKYLD(Aib)RRAQAFVK(4-Pal)LLST-OH (SEQ ID NO:4).

8. A compound of claim 4 which is
HS[Dab(Ac)]GTFTSDYSKYLD(Aib)RRAQAFVK(4-Pal)LLST-OH (SEQ ID NO:5).

9. A compound of claim 5 which is
HS[Dab(Ac)]GTFTSDYSKYLD(Aib)RRAQQFVK(4-Pal)LLST-OH (SEQ ID NO:6).

10. A pharmaceutical composition comprising the compound of any one of claims 1 to 9 and a pharmaceutically acceptable buffer.

11. The pharmaceutical composition of claim 10, wherein the pharmaceutically acceptable buffer is histidine-buffered saline.

12. The pharmaceutical composition of claim 10 or claim 11 further comprising an additional therapeutic agent.

13. The pharmaceutical composition of claim 12, wherein the additional therapeutic agent is insulin.

14. A compound according to any one of claims 1 to 9 or a pharmaceutical composition according to any one of claims 10 to 13 for use in a therapy,

15. A compound according to any one of claims 1 to 9 or a pharmaceutical composition according to any one of claims 10 to 13 for use in the treatment of hypoglycemia.

16. A compound or pharmaceutical composition for use according to claim 14 or claim 15 wherein the compound is administered with an additional therapeutic agent.

17. A compound or pharmaceutical composition for use according to claim 16, wherein the additional therapeutic agent is an insulin.

## Patentansprüche

1. Verbindung, die eine Aminosäuresequenz nach HSX₁GTFTSDYSKYLD(Aib) RRAQX₂FVK(4-Pal)LLST umfasst, wobei X₁ für Q oder Dab(Ac) steht und X₂ für Q oder A steht (SEQ ID Nr.: 2).

2. Verbindung nach Anspruch 1, wobei X₁ für Q steht und X₂ für Q steht (SEQ ID Nr.: 3).

3. Verbindung nach Anspruch 1, wobei X₁ für Q steht und X₂ für A steht (SEQ ID Nr.: 4).

4. Verbindung nach Anspruch 1, wobei X₁ für Dab(Ac) steht und X₂ für A steht (SEQ ID Nr.: 5).

5. Verbindung nach Anspruch 1, wobei X₁ für Dab(Ac) steht und X₂ für Q steht (SEQ ID Nr.: 6).

6. Verbindung nach Anspruch 2, wobei es sich um HSQGTFTSDYSKYLD(Aib)RRAQQFVK(4-Pal)LLST-OH handelt (SEQ ID Nr.: 3).

7. Verbindung nach Anspruch 3, wobei es sich um HSQGTFTSDYSKYLD(Aib)RRAQAFVK(4-Pal)LLST-OH handelt (SEQ ID Nr.: 4).

8. Verbindung nach Anspruch 4, wobei es sich um HS[Dab(Ac)]GTFTSDYSKYLD(Aib)RRAQAFVK(4-Pal)LLST-OH handelt (SEQ ID Nr.: 5).

9. Verbindung nach Anspruch 5, wobei es sich um HS[Dab(Ac)]GTFTSDYSKYLD(Aib)RRAQQFVK(4-Pal)LLST-OH handelt (SEQ ID Nr.: 6).

10. Pharmazeutische Zusammensetzung, welche die Verbindung nach einem beliebigen der Ansprüche 1 bis 9 sowie einen pharmazeutisch unbedenklichen Puffer umfasst.

11. Pharmazeutische Zusammensetzung nach Anspruch 10, wobei es sich bei dem pharmazeutisch unbedenklichen Puffer um histidingepufferte Kochsalzlösung handelt.

12. Pharmazeutische Zusammensetzung nach Anspruch 10 oder 11, die weiterhin ein zusätzliches therapeutisches Mittel umfasst.

13. Pharmazeutische Zusammensetzung nach Anspruch 12, wobei es sich bei dem zusätzlichen therapeutischen Mittel um Insulin handelt.

14. Verbindung nach einem beliebigen der Ansprüche 1 bis 9 oder pharmazeutische Zusammensetzung nach einem beliebigen der Ansprüche 10 bis 13 zur therapeutischen Verwendung.

15. Verbindung nach einem beliebigen der Ansprüche 1 bis 9 oder pharmazeutische Zusammensetzung nach einem beliebigen der Ansprüche 10 bis 13 zur Verwendung bei der Behandlung von Hypoglykämie.

16. Verbindung oder pharmazeutische Zusammensetzung zur Verwendung nach Anspruch 14 oder Anspruch 15, wobei die Verbindung mit einem zusätzlichen therapeutischen Mittel verabreicht wird.

17. Verbindung oder pharmazeutische Zusammensetzung zur Verwendung nach Anspruch 16, wobei es sich bei dem zusätzlichen therapeutischen Mittel um ein Insulin handelt.

## Revendications

1. Composé comprenant une séquence d'acides aminés de HSX₁GTFTSDYSKYLD(Aib) RRAQX₂FVK(4-Pal)LLST, dans lequel X₁ représente Q ou Dab(Ac) et X₂ représente Q ou A (SEQ ID NO: 2).

2. Composé selon la revendication 1, dans lequel X₁ représente Q et X₂ représente Q (SEQ ID NO: 3).

3. Composé selon la revendication 1, dans lequel X₁ représente Q et X₂ représente A (SEQ ID NO: 4).

4. Composé selon la revendication 1, dans lequel X₁ représente Dab(Ac) et X₂ représente A (SEQ ID NO: 5).

5. Composé selon la revendication 1, dans lequel X₁ représente Dab(Ac) et X₂ représente Q (SEQ ID NO: 6).

6. Composé selon la revendication 2, à savoir HSQGTFTSDYSKYLD(Aib)RRAQQFVK(4-Pal)LLST-OH (SEQ ID NO: 3).

7. Composé selon la revendication 3, à savoir HSQGTFTSDYSKYLD(Aib)RRAQAFVK(4-Pal)LLST-OH (SEQ ID NO: 4).

8. Composé selon la revendication 4, à savoir HS[Dab(Ac)]GTFTSDYSKYLD(Aib)RRAQAFVK(4-Pal)LLST-OH (SEQ ID NO: 5).

9. Composé selon la revendication 5, à savoir HS[Dab(Ac)]GTFTSDYSKYLD(Aib)RRAQQFVK(4-Pal)LLST-OH (SEQ ID NO: 6).

10. Composition pharmaceutique qui comprend le composé selon l'une quelconque des revendications 1 à 9 ainsi qu'un tampon pharmaceutiquement acceptable.

11. Composition pharmaceutique selon la revendication 10, dans laquelle le tampon pharmaceutiquement acceptable représente une solution saline tamponnée à l'histidine.

12. Composition pharmaceutique selon la revendication 10 ou la revendication 11, qui comprend en outre un agent thérapeutique supplémentaire.

13. Composition pharmaceutique selon la revendication 12, dans laquelle l'agent thérapeutique supplémentaire est l'insuline.

14. Composé selon l'une quelconque des revendications 1 à 9 ou composition pharmaceutique selon l'une quelconque des revendications 10 à 13 pour son utilisation en thérapie.

15. Composé selon l'une quelconque des revendications 1 à 9 ou composition pharmaceutique selon l'une quelconque des revendications 10 à 13 pour son utilisation dans le traitement de l'hypoglycémie.

16. Composé ou composition pharmaceutique pour son utilisation selon la revendication 14 ou la revendication 15, dans lequel ou dans laquelle le composé est administré avec un agent thérapeutique supplémentaire.

17. Composé ou composition pharmaceutique pour son utilisation selon la revendication 16, dans lequel ou dans laquelle l'agent thérapeutique supplémentaire est une insuline.
